# EUROPEAN PATENT SPECIFICATION

(11) **EP 2 986 715 B1**
(45) Date of publication and mention of the grant of the patent: **22.08.2018**
(21) Application number: 14785372.5
(22) Date of filing: 17.04.2014
(51) Int. Cl.: C12N 5/079, C12N 5/0793, C12N 5/0797

(54) **MEDIA COMPOSITIONS FOR NEURONAL CELL CULTURE**
MEDIENZUSAMMENSETZUNGEN FÜR NEURONALE ZELLKULTUR
COMPOSITIONS DE MILIEUX DE CULTURE DE CELLULES NEURONALES

(30) Priority: 17.04.2013 US 201361813034 P
(43) Date of publication of application: 24.02.2016
(73) Proprietor: Salk Institute for Biological Studies, La Jolla, CA 92037-1002 (US)
(72) Inventor: GAGE, Fred, H., La Jolla, CA 92037 (US); BARDY, Cedric, Del Mar, CA 92014 (US)
(74) Representative: Barker Brettell LLP
(86) International application number: PCT/US2014/034565
(87) International publication number: WO 2014/172580

(56) References cited:
- WO-A1-97/38090
- WO-A2-2012/174225
- US-A1- 2008 160 006
- US-A1- 2012 052 577
- Anonymous: "ACSF (Artificial cerebrospinal fluid)", , 1 January 2007 (2007-01-01), XP055295715, Retrieved from the Internet: URL:http://cshprotocols.cshlp.org/content/ 2007/2/pdb.rec10804.full?text_only=true [retrieved on 2016-08-16]
- Leah F. Boyer ET AL: "Dopaminergic Differentiation of Human Pluripotent Cells" In: "Current Protocols in Stem Cell Biology", 1 August 2012 (2012-08-01), John Wiley & Sons, Inc., Hoboken, NJ, USA, XP055295978, ISBN: 978-0-470-15180-8 DOI: 10.1002/9780470151808.sc01h06s22, * the whole document *
- M. M.A.J J C R.V F.H L L V S C A.G T B M J Hurk Gorris Brown Bardy Hernandez Gage Boyer Bohnke Palomares Simon Marchand Bang Eames: "Neuronal medium that supports basic synaptic functions and activity of human neurons in vitro", Proceedings of the National Academy of Science of the United States of America, 13 April 2015 (2015-04-13), pages E2725-34, XP055295336, United States DOI: 10.1073/pnas.1504393112 Retrieved from the Internet: URL:http://www.pnas.org/content/112/20/E27 25.full.pdf?with-ds=yes [retrieved on 2016-08-12]
- SPILIOTOPOULOS, D. ET AL.: 'An optimized experimental strategy for efficient conversion of embryonic stem (ES) -derived mouse neural stem (NS) cells into a nearly homogeneous mature neuronal population' NEUROBIOLOGY OF DISEASE vol. 34, 2009, pages 320 - 331, XP026052965
- KARUMBAYARAM, S. ET AL.: 'Directed differentiation of human-induced pluripotent stem cells generates active motor neurons' STEM CELLS vol. 27, 2009, pages 806 - 811, XP003030980

## Description

### BACKGROUND OF THE INVENTION

Substantial hopes and efforts are drawn into stem cell and somatic cell reprogramming to model human diseases and find urgently needed therapies. The recent Nobel prize in Medicine and Physiology awarded to Dr. Yamanaka only 5 years after publishing a revolutionary method to reprogram human somatic cells into pluripotent stem cells confirm how much hope is invested in Induced Pluripotent Stem Cell (IPSC) technology (Takahashi *et al.,* 2007). Shortly after, it was shown that IPSC derived from skin cells of patients could be turned into neurons in vitro (Dimos *et al.,* 2008). Neuroscientists rushed on this unprecedented opportunity to obtain culture of live neurons in vitro from patients with neurological and psychiatric disorders and demonstrated that major phenotypes associated with particular disorders could be recapitulated in the dish (Marchetto *et al.,* 2010; Brennand *et al.,* 2011; Marchetto *et al.,* 2011; Nguyen *et al.,* 2011; Seibler *et al.,* 2011; Bellin *et al.,* 2012; Egawa *et al.,* 2012; Israel *et al.,* 2012; Shi *et al.,* 2012).

Yet the basic culture conditions to grow neurons in vitro have never been established with a thorough neurophysiological approach. Currently almost all human neuronal culture are grown in Dulbecco Modified Eagle Media (DMEM) (Cattaneo and McKay, 1990; Okabe *et al.,* 1996; Soldner *et al.,* 2009; Vierbuchen *et al.,* 2010; Brennand *et al.,* 2011; Caiazzo *et al.,* 2011; Eiraku *et al.,* 2011; Marchetto *et al.,* 2011; Nguyen *et al.,* 2011; Pang *et al.,* 2011; Pfisterer *et al.,* 2011; Qiang *et al.,* 2011; Soldner *et al.,* 2011; Son *et al.,* 2011; Boyer *et al.,* 2012; Bráz *et al.,* 2012; Giorgetti *et al.,* 2012; Israel *et al.,* 2012; Shao *et al.,* 2012; Vilchez *et al.,* 2012), and occasionally in Neurobasal media (Hermann *et al.,* 2004; Li *et al.,* 2005; Johnson *et al.,* 2007) or a mixture of both DMEM and Neurobasal (Koch *et al.,* 2009; Boulting *et al.,* 2011; Kriks *et al.,* 2011; Egawa *et al.,* 2012; Shi *et al.,* 2012). Then a variety of growth factors and supplements are added to the basal media to promote neuronal differentiation and survival. Using patch-clamping and calcium imaging techniques Applicants found that those widely used basal media are extremely unadapted for neurophysiological function. They strongly impair action potential firing as well as excitatory and inhibitory synaptic function, which therefore dramatically reduces spontaneous neural activity. This in turn has detrimental consequences on the functional development and operation of neurons in vitro. Thus, there is a need in the art for media compositions that closely reflect in vivo physiological conditions to provide for uncompromised conditions during in vitro differentiation, expansion or maintenance of, for example, mature neural cells, neural progenitor cells or primary neural cells.

US Publication No. 2008/160006 discloses a method to improve neural cell viability in brain or spinal cord tissue after brain or spinal cord injury including applying a sterile liquid medium to the brain or spinal cord tissue.

US Publication No. 2012/052577 discloses methods and compositions for culturing neural stem cells and inducing their specification to the oligodendrocyte phenotype.

Spiliotopoulos *et al.,* 2009 discloses methods for differentiating ES-derived neural stem cells into neurons.

Karumbayaram *et al.,* 2009 discloses methods for producing motor neuron progenitors and motor neurons from human iPS cells.

Provided herein are, inter alia, are media compositions useful for culturing neural cells. In particular, the compositions provided herein mimic important physiological conditions in the living brain and sustain neural activity. In some embodiments, the media compositions provided herein improve the efficiency of human neuron differentiation and promote synaptic function in long-term in vitro cultures.

### SUMMARY OF THE INVENTION

The present application provides for a cell medium that promotes the growth and/or maintenance and/or functional activity of brain cells cultured in the medium. In certain embodiments, the cell medium comprises one or more neuroactive inorganic salt, and/or one or more neuroactive amino acid, and/or one or more vitamin, and/or one or more amino acid, and/or one or more energetic substrate, and/or one or more pH modulating agent.

In certain embodiments, the medium comprises sodium chloride at a concentration of between 70 and 150 mM; a neuroactive inorganic salt at a concentration of between 0.000001 and 10 mM, selected from the group consisting of consisting of Potassium Chloride, Calcium Chloride, Magnesium Sulfate, Magnesium Chloride, Ferric Nitrate, Zinc sulfate, Cupric sulfate, Ferric sulfate, and combinations thereof; Glycine at a concentration of between 0.0001 and 0.05 mM; L-alanine at a concentration of between 00001 and 0.05 mM; and L-serine at a concentration of between 0.001 and 0.03 mM.

In certain embodiments, the medium comprises L-alanine at a concentration below 0.05 mM, glycine at a concentration below 0.25 mM, L-serine at a concentration below 0.25 mM, L-proline at a concentration below 0.15 mM, L-arginine hydrochloride at a concentration below 0.60 mM, L-alanyl-L-glutamine at a concentration below 2.5 mM, magnesium sulfate at a concentration above 0.41 mM, calcium chloride at a concentration above 1.05 mM, potassium chloride at a concentration above 4.16 mM, sodium chloride at a concentration above 120.7 mM, D-glucose at a concentration below 17.5 mM or HEPES at a concentration of 5 mM.

In certain embodiments, the present application provides for a medium comprising one or more neuroactive inorganic salt and one or more neuroactive amino acid. In certain embodiments, the medium further comprises one or more pH modulating agent, one or more energetic substrate, one or more amino acid, and/or one or more vitamin, and combinations thereof. In certain embodiments, the ingredients of the medium are present in amounts that promote the growth, maintenance and neural functionality of a neural cell cultured in the medium.

In certain embodiments, the medium does not comprise serum.

In certain embodiments, the medium comprises one or more energy sensitive ingredient, wherein contacting the ingredient with a source of energy, for example light or electricity, increases the activity of a neural cell cultured in the medium. In certain embodiments, continued exposure of the ingredient to the energy source results in excitotoxicity of the neural cell cultured in the medium.

In another aspect, a method of culturing a neuronal cell is provided. The method includes, contacting a neuronal cell with a medium as provided herein including embodiments thereof and allowing the neuronal cell to grow, thereby culturing a neuronal cell.

In another aspect, a neuronal cell cultured in a medium as provided herein including embodiments thereof is provided.

### BRIEF DESCRIPTION OF THE DRAWINGS

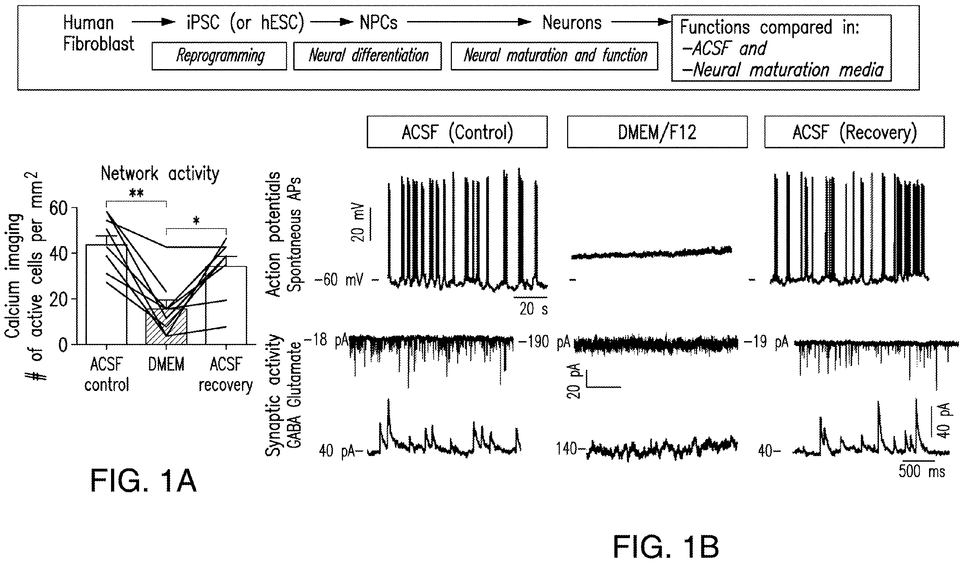

**Figure 1A****-C:** DMEM or Neurobasal based media impair neuronal and synaptic activity. Human neurons derived from iPS or ES cells were plated directly on glass coverslips. The basic neuronal functions of single mature neurons was tested and compared in two classic extracellular basal media (DMEM/F12 or Neurobasal-A). The perfusion of extracellular media was switched from ACSF to DMEM/F12 or Neurobasal-A, and back to ACSF for recovery. **A.** Calcium imaging analysis showing the number of spontaneously active cells in 10-min movies of the same field of view recorded in ACSF, DMEM/F12 and back to ACSF. Inorganic salt concentration and the osmolarity of ACSF were matched to DMEM. The gray lines represent individual fields of view. Statistics were performed with Wilcoxon test, **P =0.009; *P = 0.022). **B.** Top traces: Current-clamp recordings reveal that spontaneous action potentials observed in ACSF quickly disappear in DMEM, together with a large increase in resting membrane potential. Middle traces: Voltage-clamp recordings (at - 70 mV, Cl- reversal potential) reveal that DMEM-based medium induces a large Na+ inward current and completely blocks spontaneous AMPA synaptic activity. Bottom traces: Voltage-clamp recordings (at 0 mV, Na+ reversal potential) reveal that DMEM-based medium induces large Cl- currents and completely blocked spontaneous GABA synaptic activity. **C.** Top traces (synaptic activity): Voltage-clamp recordings reveal that Neurobasal-A based medium strongly reduces spontaneous AMPA synaptic activity. In addition, it also induces large tonic Cl-currents and completely blocks spontaneous GABA synaptic activity. Bottom traces (action potentials): NB-A does not affect the resting membrane potential but it strongly impairs evoked action potentials (500ms step of current), voltage dependent Nav/Kv currents (IV traces, clamp -70mV, voltage steps 5 mV) and spontaneous action potentials.

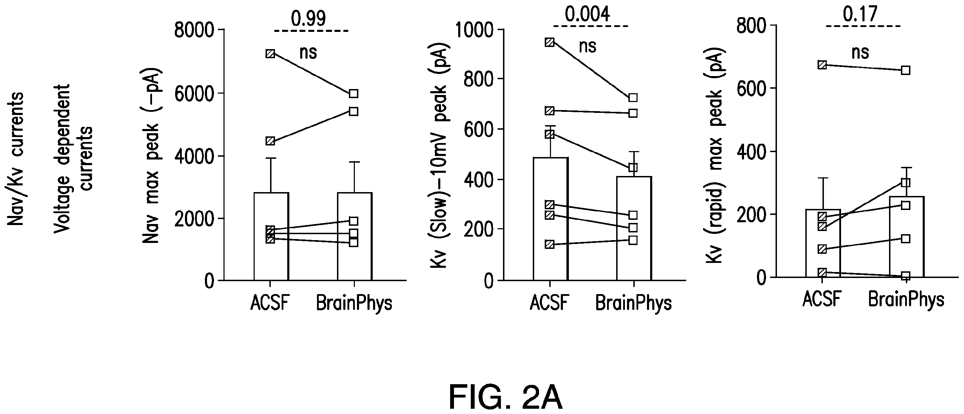

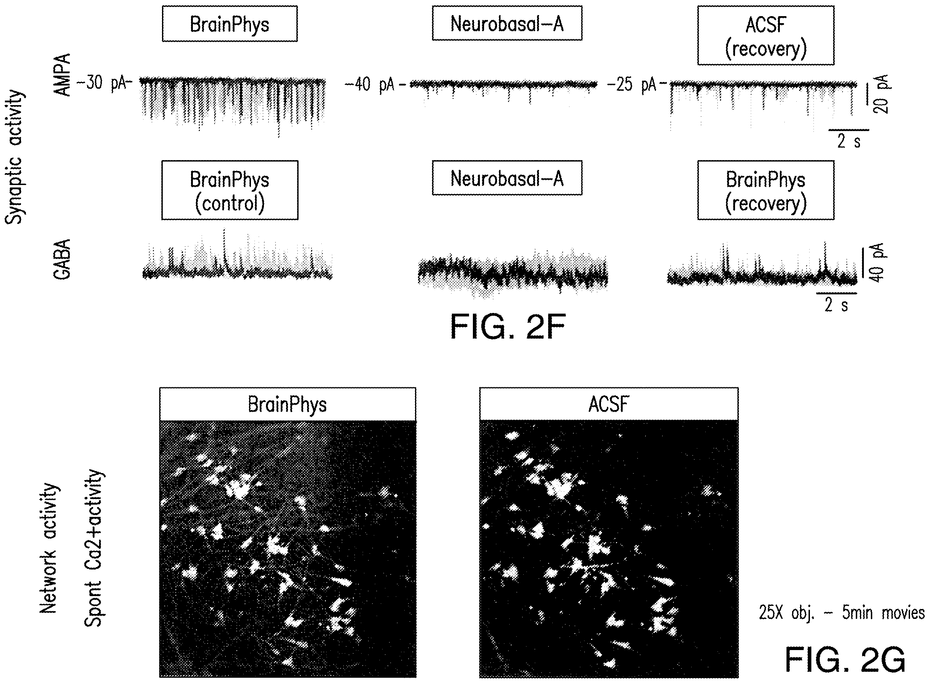

**Figure 2A****-G:** BrainPhys supports efficient action potential activity. **A.** Although the peaks of voltage-gated currents can be variable between single neurons, they are similar in BrainPhys and ACSF. Each neuron was tested both in ACSF and BrainPhys (black vs blue dots). Non-parametric paired Wilcoxon test P-values are shown in italics. Histograms represent the mean ± standard error. **B.** The resting membrane potential, spontaneous and evoked action potentials were the same in ACSF and BrainPhys. **C.** Typical mature patched neuron expressing an optogene (synapsin:Cheta-YFP) and filled with rhodamine. Optogenetic control of neuronal activity can be reliably achieved in BrainPhys. **D.** Single mature neuron tested with the same parameters in BrainPhys or Neurobasal-A illustrates that optogenetic control is dramatically improved in BrainPhys. **E.** The maximum amplitudes of voltage-gated Nav and Kv currents (measured from IV curves, clamp -70mV, steps 5 mV) of single neurons were significantly reduced in Neurobasal media (red dots = Neurobasal-A, orange dots = Neurobasal) compared to BrainPhys (blue dots) or ACSF (black dots). **F.** Both excitatory (AMPA) and inhibitory (GABA) synaptic activity was clearly apparent in BrainPhys medium. The perfusion of different media while recording the spontaneous synaptic activity of single neurons revealed that BrainPhys better support synaptic function compared the other media such as Neurobasal-A. Twelve sweeps are super-imposed in gray and one of them is highlighted in black for clarity. **G.** Spontaneous activity measured at the network level with calcium imaging in the presence of ACSF and BrainPhys.

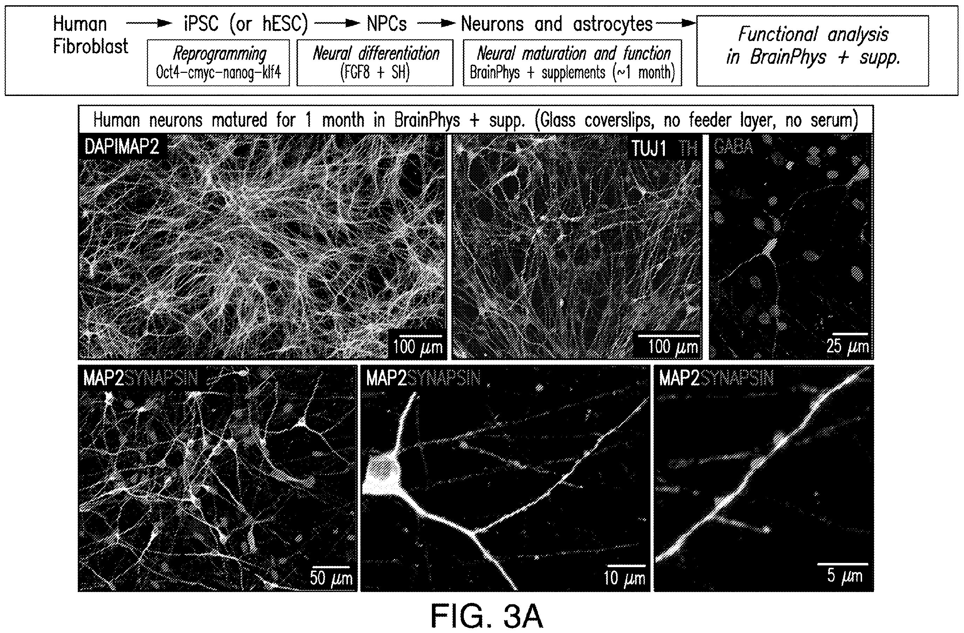

**Figure 3A****-D:** Culture of healthy, mature and active human neurons in BrainPhys based medium. **A.** Immunostaining of typical human IPSC-derived neuronal cultures grown in BrainPhys with supplements for more than 4 weeks. **B-C.** Electrophysiological activity of typical functional neurons maturing in BrainPhys + supplements for 3 to 6 weeks. Patch-clamp recordings were also performed in BrainPhys medium. **B.** From left to right: Train of action potentials evoked by a small 500 ms depolarizing step of current or brief flashes of light (syn:Cheta-YFP). IV traces (clamp -70mV, steps 5 mV) showing typical voltage dependent Na+ and potassium currents. Spontaneous action potentials recorded in current-clamp. C. Spontaneous synaptic events mediated by AMPA receptors (sensitive to NBQX, voltage clamp at -70mV) and GABA receptors (sensitive to Gabazine, voltage clamp at 0mV). **D.** Calcium imaging showing typical activity of neural networks grown and recorded in BrainPhys + supplements. Time series analysis is plotted for the active region of interest.

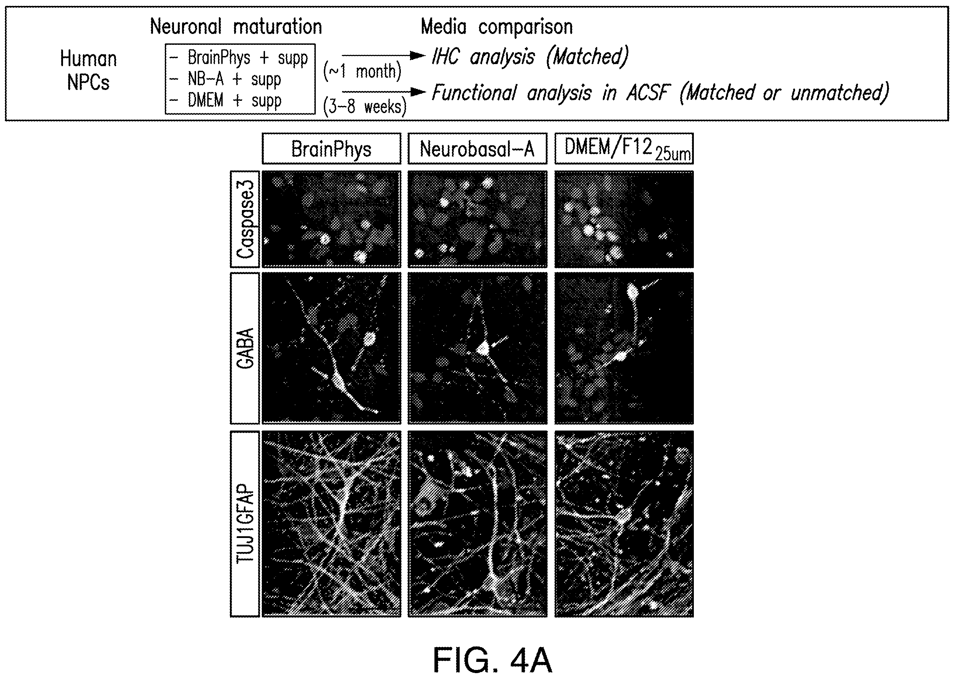

**Figure 4A****-J:** Characterization of human neurons cultured in BrainPhys medium compared to media based on DMEM/f12 or Neurobasal. **A-G.** Representative immunostaining observed in the three cultures growing side by side. Nuclei were stained with DAPI (blue). Despite some variability, no clear differences were found between survival of human neurons, the proportion of neuronal subtypes (e.g. dopaminergic, gabaergic) and the number of proximal synaptic puncta. **H.** Functional types of neurons were defined based on their firing patterns in response to 500-ms depolarizing steps of currents. **I.** To characterize the influence of BrainPhys on functional maturation, neurons were cultured in the same plates either in BrainPhys or DMEM/F12 based media. Patch clamping was blindly performed in ACSF. Higher proportions of mature Type 5 neurons were found in the BrainPhys cultures. BrainPhys culture also showed higher proportions of neurons (Type 3, 4 and 5) receiving functional AMPA synaptic inputs (NBQX sensitive) or GABA synaptic inputs (Gabazine sensitive), determined by having at least 5 spontaneous synaptic events with distinctive kinetics over a 4-min recording in voltage clamp (-70 mV or 0mV, respectively). **J.** Further analysis of a larger sample of neurons obtained from various iPS cell lines and patched in ACSF confirmed that culturing neurons in BrainPhys based medium considerably increases the chances of obtaining synaptically active neurons.

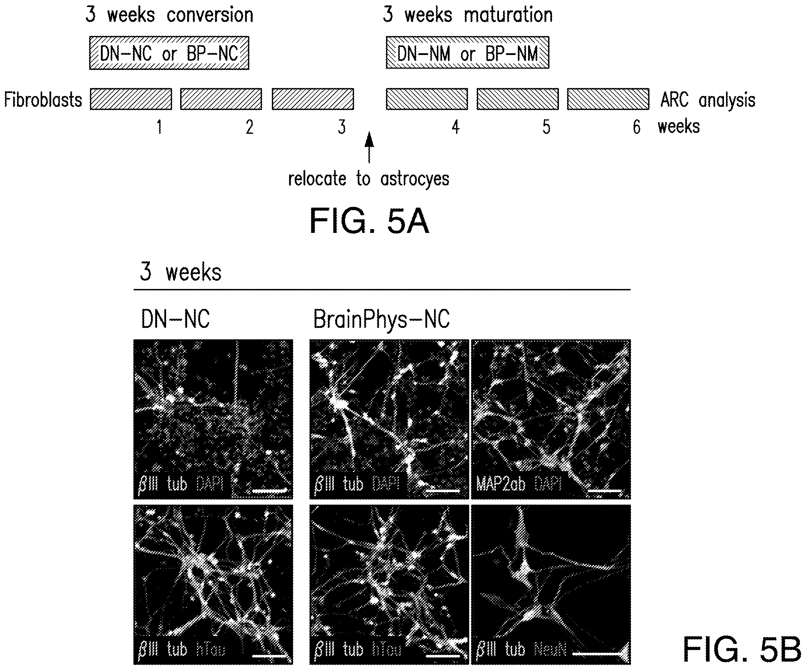

**Figure 5A****-C:** BrainPhys-based neuronal media permits efficient direct neuronal conversion (iN) as well as functional maturation of human iN cells and increases ARC protein expression. **A.** Experimental design: iN-competent HDFs were converted in NC media for three weeks. For maturation iN cells were relocated onto astrocytes and further cultured in NM media. **B.** Immunofluorescence staining for beta-III-tubulin, hTau, MAP2ab and NeuN following 3 weeks conversion. Scale: 50 µm. **C.** Representative image of immunofluorescence staining for hTau and ARC in 6-weeks-old iN cells following maturation on astrocytes and analysis. Quantification using ROI selection and measurement in ImageJ. Scale: 100 µm. Quantification of neuronal ARC levels in iN cultures derived from 3 donors. Grey points represent individual cells (average n=38 per group, minimum n=19 per group). Bar show means ± SEM of each group. *P < 0.05, **P < 0.01, ***P < 0.001, ****P < 0.0001.

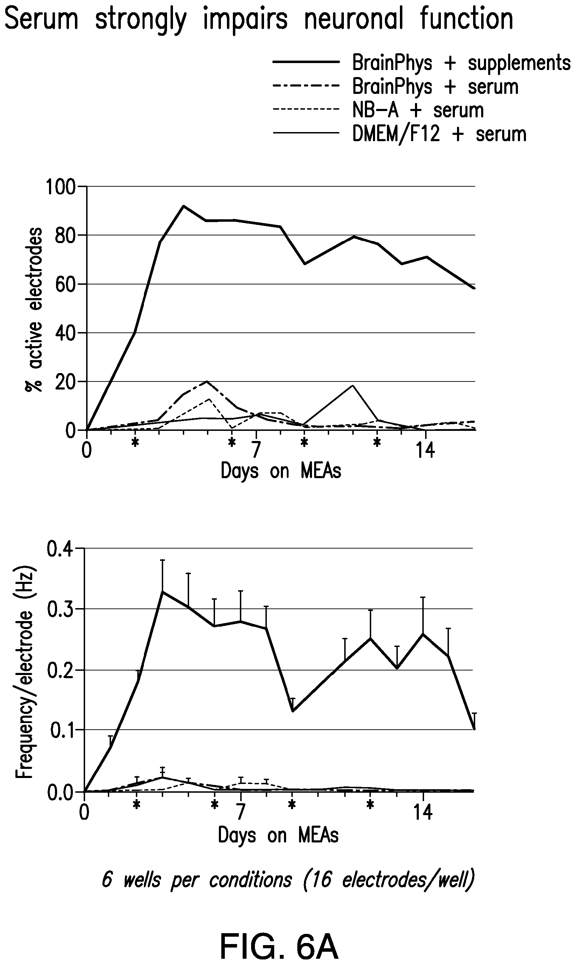

**Figure 6A****-C:** Shows testing of electrical activity of human neurons in BrainPhys over several weeks using a multi-electrode array (MEA) and compared to currently used conditions. BrainPhys improves longterm neuronal activity. **A.** Adding serum (e.g. knockout serum, fetal bovine serum FBS) to maturation medium strongly impair neuronal function. **B.** BrainPhys reduces variability due to feed cycles and improves and maintains longterm neuronal function compared to tested NB/DMEMF12 mixture or DMEM/F12. **C.** BrainPhys supplemented but without serum improve and sustain neuronal activity for several weeks compared to iCell neuron medium or Neurobasal with identical serum-free supplements.

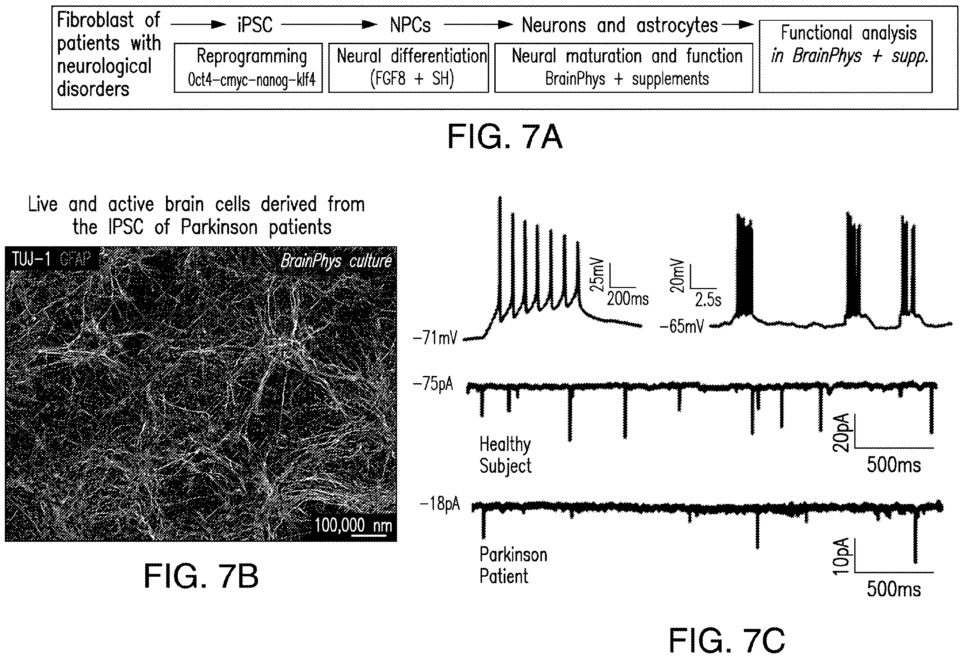

**Figure 7A****-C:** Shows the activity of Parkinson patients' iPSC-derived neurons cultured in BrainPhys + supplements. **A.** Diagram showing generation of iPSC-derived neurons. **B.** Live and active brain cells derived from the iPSC of Parkinson patients stained for TUJ-1 and GFAP after culture in BrainPhys. **C.** Activity of neural cells from healthy subjects and Parkinson patients.

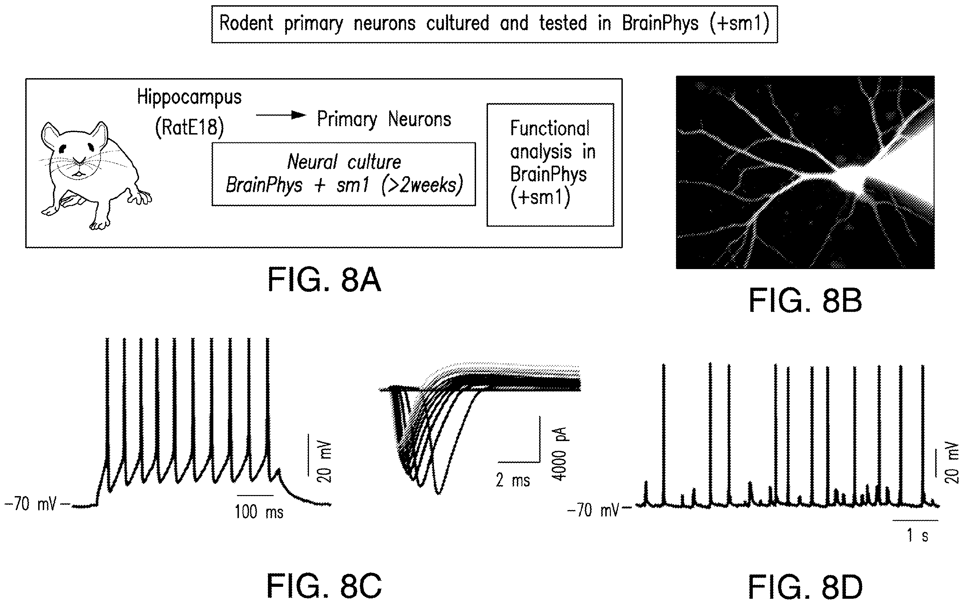

**Figure 8A****-G:** Differentiation of healthy and active rat primary hippocampal neurons in BrainPhys. **A-G.** Electrophysiological activity of typical functional neurons grown in BrainPhys + supplement (sm1) for 2-3 weeks. **A.** Diagram showing that primary neurons from rat hippocampus were cultured in BrainPhys + sm1 for greater than 2 weeks, and then functionally analyzed. **B.** Image of a typical patch-clamped neuron filled with Rhodamine. On the right, the bright white shadow is the Rhodamine-filled patch pipette. **C.** Train of action potentials evoked by a small 500-ms depolarizing step of current, and IV traces (clamp -70 mV, steps 5 mV) showing typical voltage-dependent Na+ and K+ currents. Colored traces correspond to different voltage steps. **D.** Spontaneous action potentials recorded in current clamp. **E.** Spontaneous GABA synaptic events recorded in voltage clamp at (0 mV). **F.** Spontaneous AMPA synaptic events recorded in voltage clamp (-70 mV). **G.** Coverslips were fixed after patching and processed for immunohistochemistry. Dendrites were stained with MAP2, presynaptic terminals with Synapsin and nuclei with DAPI.

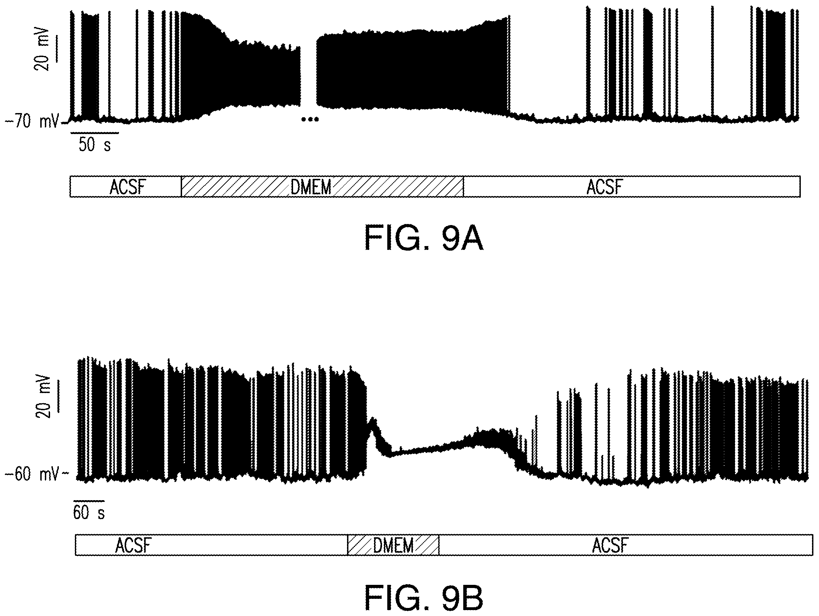

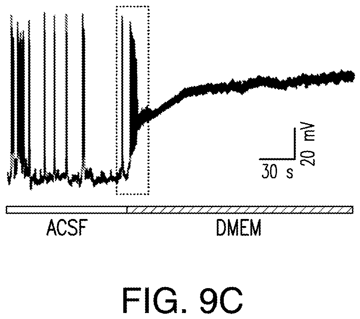

**Figure 9A****-C:** DMEM depolarized the membrane potential of every examined neuron, and in most cases, it also saturated and silenced spontaneous action potential firing. The traces were obtained from whole cell current clamp spontaneous recordings of stem cell-derived human neurons in ACSF or DMEM. **A.** Rare example in which DMEM did not saturate and silence action potential firing. **B-C.** Two more typical examples, where DMEM-induced depolarization silenced spontaneous action potential firing.

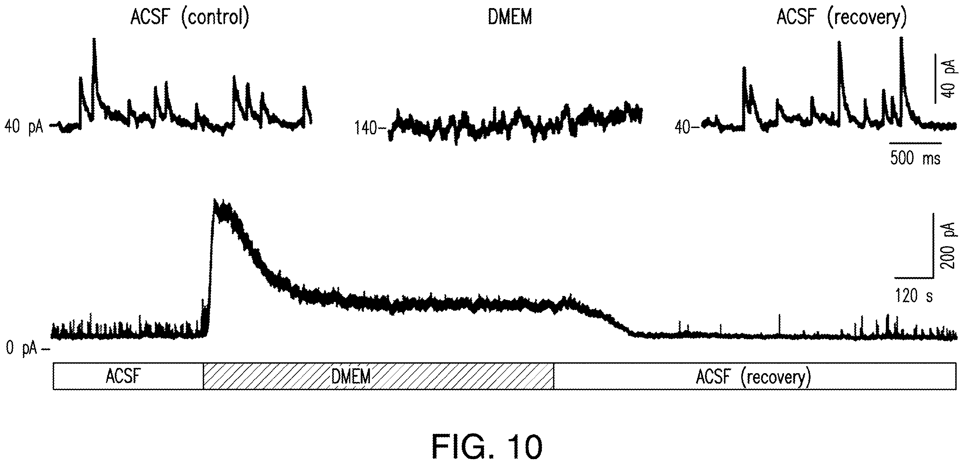

**Figure 10****:** DMEM induces large Cl- currents and blocks spontaneous inhibitory synaptic activity. Voltage-clamp recording (0 mV) of a typical neuron showing spontaneous GABA synaptic activity in ACSF that could be blocked by DMEM and recovered. Top traces represent typical GABA events, which were seen in ACSF but not in DMEM. Bottom trace is the corresponding continuous recording while perfusion was alternated, showing a large Cl- influx at the onset of DMEM perfusion followed by a plateau.

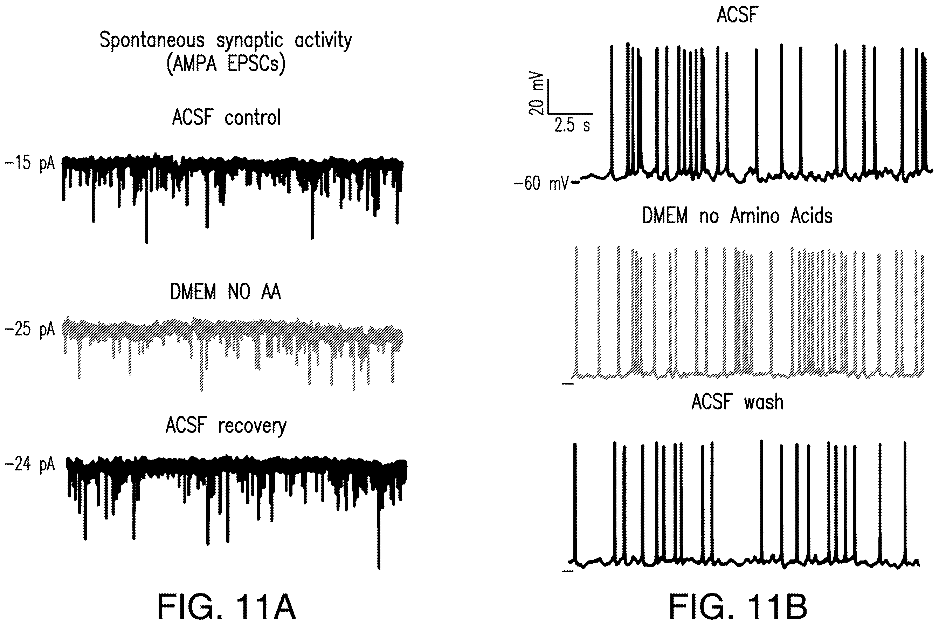

**Figure 11A****-D:** DMEM in which all the vitamins, all the amino acids or all the extra components of the media were removed. By removing the bulk of the amino acids, the DMEM-induced depolarizations that caused the impairment of action potentials and synaptic activity in DMEM was avoided. However, this solution failed to sustain the development and survival of the neurons for more than a week.

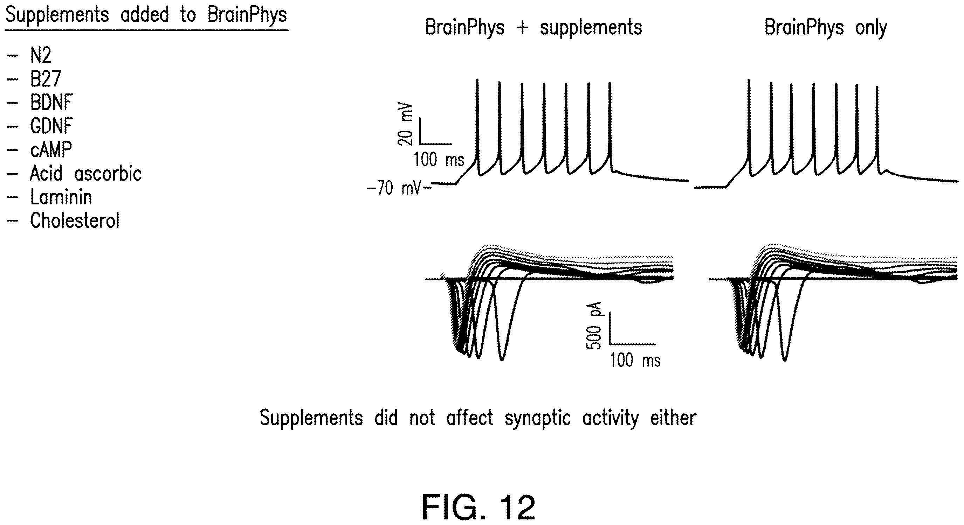

**Figure 12****:** Acutely adding a complete set of commonly used supplements (N2, B27, Retinoic acid, BDNF, GDNF, ascorbic acid, cAMP, laminin, and cholesterol) to BrainPhys basal medium did not affect the firing rate or excitatory/inhibitory synaptic activity.

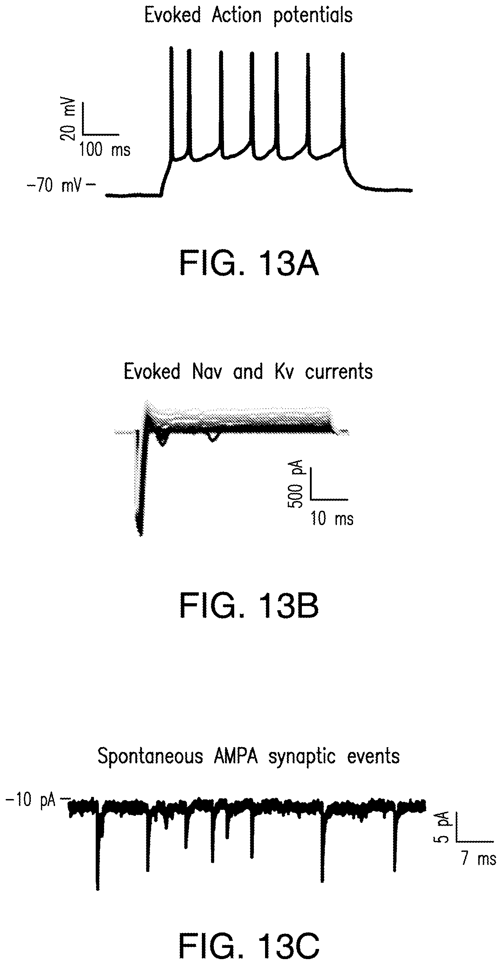

**Figure 13A-C.** Patch clamp recordings of mouse brain slices (*ex vivo*) in BrainPhys. **A-C.** Typical functional granule neuron patched from the adult mouse hippocampus in BrainPhys. **A.** Action potentials evoked with a 500-ms step of current from resting membrane potential. **B.** IV traces of incremental 5-mV steps (rest -70 mV) showing Nav and Kv currents. **C.** Spontaneous AMPA synaptic events (voltage clamp -70 mV).

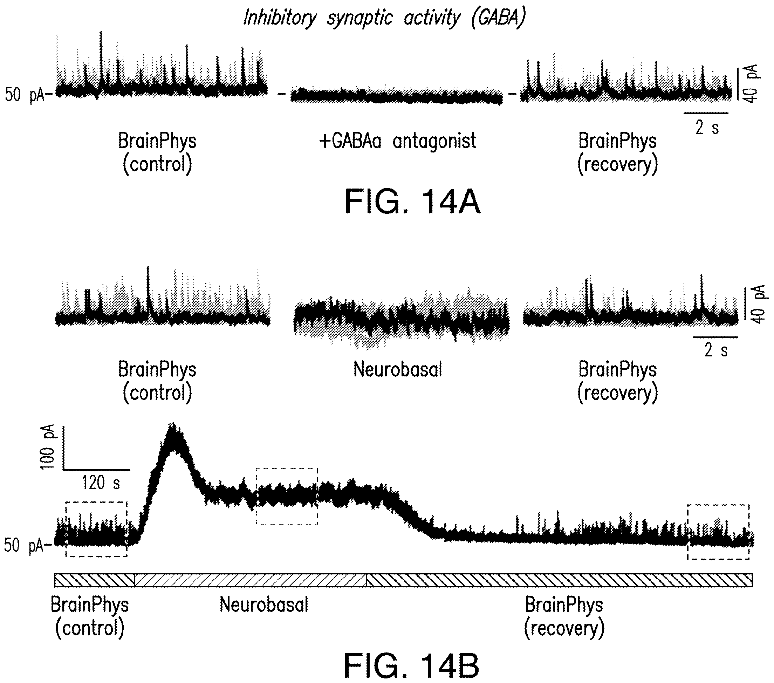

**Figure 14A****-B:** Neurobasal induces large Cl- currents and blocks spontaneous inhibitory synaptic activity.

### DETAILED DESCRIPTION OF THE INVENTION

The present disclosure is based, at least in part, on the discovery of a medium that promotes the growth and maintenance, as well as the functional activity, of neural cells cultured in the medium. In particular, the application discloses that such medium can comprise a combination of neuroactive inorganic salts, neuroactive amino acids, pH modulating agents, energetic substrates, amino acids, and vitamins. In certain embodiments, the ingredients of the medium are present in amounts that promote the growth, maintenance and neural functionality of a neural cell cultured in the medium.

### I. Definitions

Unless defined otherwise, technical and scientific terms used herein have the same meaning as commonly understood by a person of ordinary skill in the art. See, *e.g.,* Singleton et al., DICTIONARY OF MICROBIOLOGY AND MOLECULAR BIOLOGY 2nd ed., J. Wiley & Sons (New York, NY 1994); Sambrook et al., MOLECULAR CLONING, A LABORATORY MANUAL, Cold Springs Harbor Press (Cold Springs Harbor, NY 1989). Any methods, devices and materials similar or equivalent to those described herein can be used in the practice of this invention. The following definitions are provided to facilitate understanding of certain terms used frequently herein and are not meant to limit the scope of the present disclosure.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally occurring amino acid, *i.e.,* an α carbon that is bound to a hydrogen, a carboxyl group, an amino group, and an R group, *e.g*., homoserine, norleucine, methionine sulfoxide, methionine methyl sulfonium. Such analogs have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid. The terms "non-naturally occurring amino acid" and "unnatural amino acid" refer to amino acid analogs, synthetic amino acids, and amino acid mimetics which are not found in nature.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

A "cell culture" is an in vitro population of cells residing outside of an organism. The cell culture can be established from primary cells isolated from a cell bank or animal, or secondary cells that are derived from one of these sources and immortalized for long-term *in vitro* cultures.

The terms "culture," "culturing," "grow," "growing," "maintain," "maintaining," "expand," "expanding," etc., when referring to cell culture itself or the process of culturing, can be used interchangeably to mean that a cell is maintained outside the body (*e.g., ex vivo*) under conditions suitable for survival. Cultured cells are allowed to survive, and culturing can result in cell growth, differentiation, or division. The term does not imply that all cells in the culture survive or grow or divide, as some may naturally senesce, etc. Cells are typically cultured in media, which can be changed during the course of the culture.

The terms "medium," "media" and "culture solution" refer to the cell culture milieu. Media is typically an isotonic solution, and can be liquid, gelatinous, or semisolid, *e.g.,* to provide a matrix for cell adhesion or support. Media, as used herein, can include the components for nutritional, chemical, and structural support necessary for culturing a cell.

As used herein, "conditions to allow growth" in culture and the like refers to conditions of temperature (typically at about 37° C for mammalian cells), humidity, CO₂ (typically around 5%), in appropriate media (including salts, buffer, serum), such that the cells are able to undergo cell division or at least maintain viability for at least 24 hours, preferably longer (*e.g.,* for days, weeks or months).

The term "derived from," when referring to cells or a biological sample, indicates that the cell or sample was obtained from the stated source at some point in time. For example, a cell derived from an individual can represent a primary cell obtained directly from the individual (*i.e.,* unmodified), or can be modified, *e.g.,* by introduction of a recombinant vector, by culturing under particular conditions, or immortalization. In some cases, a cell derived from a given source will undergo cell division and/ or differentiation such that the original cell is no longer exists, but the continuing cells will be understood to derive from the same source.

The term "recombinant" when used with reference, *e.g.,* to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all. Transgenic cells and animals are those that express a heterologous gene or coding sequence, typically as a result of recombinant methods.

A "somatic cell" is a cell forming the body of an organism. Somatic cells include cells making up organs, skin, blood, bones and connective tissue in an organism, but not germ line cells.

A "stem cell" is a cell characterized by the ability of self-renewal through mitotic cell division and the potential to differentiate into a tissue or an organ. Among mammalian stem cells, embryonic and somatic stem cells can be distinguished. Embryonic stem cells reside in the blastocyst and give rise to embryonic tissues, whereas somatic stem cells reside in adult tissues for the purpose of tissue regeneration and repair.

The term "pluripotent" or "pluripotency" refers to cells with the ability to give rise to progeny that can undergo differentiation, under appropriate conditions, into cell types that collectively exhibit characteristics associated with cell lineages from the three germ layers (endoderm, mesoderm, and ectoderm). Pluripotent stem cells can contribute to tissues of a prenatal, postnatal or adult organism. A standard art-accepted test, such as the ability to form a teratoma in 8-12 week old SCID mice, can be used to establish the pluripotency of a cell population. However, identification of various pluripotent stem cell characteristics can also be used to identify pluripotent cells.

An "induced pluripotent stem cell" or "iPSC" refers to a pluripotent stem cell artificially (*e.g*. non- naturally, in a laboratory setting) derived from a non-pluripotent cell. A "non-pluripotent cell" can be a cell of lesser potency to self-renew and differentiate than a pluripotent stem cell. Cells of lesser potency can be, but are not limited to adult stem cells, tissue specific progenitor cells, primary or secondary cells. An adult stem cell is an undifferentiated cell found throughout the body after embryonic development. Adult stem cells multiply by cell division to replenish dying cells and regenerate damaged tissue. Adult stem cells have the ability to divide and create another like cell and also divide and create a more differentiated cell. Even though adult stem cells are associated with the expression of pluripotency markers such as Rex1, Nanog, Oct4 or Sox2, they do not have the ability of pluripotent stem cells to differentiate into the cell types of all three germ layers. Adult stem cells have a limited potency to self-renew and generate progeny of distinct cell types. Without limitation, an adult stem cell can be a hematopoietic stem cell, a cord blood stem cell, a mesenchymal stem cell, an epithelial stem cell, a skin stem cell or a neural stem cell. A tissue specific progenitor refers to a cell devoid of self-renewal potential that is committed to differentiate into a specific organ or tissue. A primary cell includes any cell of an adult or fetal organism apart from egg cells, sperm cells and stem cells. Examples of useful primary cells include, but are not limited to, skin cells, bone cells, blood cells, cells of internal organs and cells of connective tissue. A secondary cell is derived from a primary cell and has been immortalized for long-lived in vitro cell culture.

The term "reprogramming" refers to the process of dedifferentiating a non-pluripotent cell (*e.g.,* an origin cell) into a cell exhibiting pluripotent stem cell characteristics (*e.g*., a human induced pluripotent stem cell).

Where appropriate the expanding transfected derived stem cell may be subjected to a process of selection. A process of selection may include a selection marker introduced into a an induced pluripotent stem cell upon transfection. A selection marker may be a gene encoding for a polypeptide with enzymatic activity. The enzymatic activity includes, but is not limited to, the activity of an acetyltransferase and a phosphotransferase. In some embodiments, the enzymatic activity of the selection marker is the activity of a phosphotransferase. The enzymatic activity of a selection marker may confer to a transfected induced pluripotent stem cell the ability to expand in the presence of a toxin. Such a toxin typically inhibits cell expansion and/or causes cell death. Examples of such toxins include, but are not limited to, hygromycin, neomycin, puromycin and gentamycin. In some embodiments, the toxin is hygromycin. Through the enzymatic activity of a selection maker a toxin may be converted to a non-toxin, which no longer inhibits expansion and causes cell death of a transfected induced pluripotent stem cell. Upon exposure to a toxin a cell lacking a selection marker may be eliminated and thereby precluded from expansion.

Identification of the induced pluripotent stem cell may include, but is not limited to the evaluation of the afore mentioned pluripotent stem cell characteristics. Such pluripotent stem cell characteristics include without further limitation, the expression or non-expression of certain combinations of molecular markers. Further, cell morphologies associated with pluripotent stem cells are also pluripotent stem cell characteristics.

The term "hiPSC-derived neural cell" refers to a neural progenitor cell (NPC) or a mature neuron that has been derived (*e.g*., differentiated) from a hiPSC cell in vitro. The hiPSCs can be differentiated by any appropriate method known in the art (*e.g.,* Marchetto, M. C. et al., Cell Stem Cell, 3, 649-657 (2008); Yeo, G. W. et al., PLoS Comput Biol, 3, 1951-1967 (2007)).

A neural progenitor is a cell that has a tendency to differentiate into a neural cell and does not have the pluripotent potential of a stem cell. A neural progenitor is a cell that is committed to the neural lineage and is characterized by expressing one or more marker genes that are specific for the neural lineage. Examples of neural lineage marker genes are N-CAM, the intermediate-filament protein nestin, SOX2, vimentin, A2B5, and the transcription factor PAX-6 for early stage neural markers (*i.e.* neural progenitors); NF-M, MAP-2AB, synaptosin, glutamic acid decarboxylase, βIII-tubulin and tyrosine hydroxylase for later stage neural markers (*i.e.* differentiated neural cells). Neural differentiation may be performed in the absence or presence of co-cultured astrocytes. The terms "neural" and "neuronal" are used according to their common meaning in the art and can be used interchangeably throughout.

The term "DMEM" as used herein refers to Dulbecco's Modified Eagle Medium (commercially made available by Life Technologies™) a nutrient media comprising various components including without limitation inorganic salts, amino acids, vitamins and other protein components.

A "modified DMEM" medium as referred to herein is a nutrient media comprising one or more (*e.g.,* 1, 2, 3, 4, 5, 6, 7, 8, 9, or 10) components present in DMEM. In some embodiments, the components are present at the same concentration as in DMEM. In other embodiments, the components are present at a different concentration compared to DMEM. In some embodiments, the components are present at a higher concentration relative to DMEM. In other embodiments, the components are present at a lower concentration relative to DMEM.

"HEPES" as provided herein refers to 2-[4-(2-hydroxyethyl)piperazin-1-y1]ethanesulfonic acid and is a zwitterionic organic buffering agent widely used in cell culture. HEPES refers, in the customary sense, to CAS Registry No.7365-45-9.

The term "neuroactive" as used herein refers to an agent, such as, for example, an inorganic salt or amino acid that acutely affects the neural activity of a cell. For example, a neural cell cultured in a medium comprising neuroactive agents will exhibit measurable neural activity (e.g. electrophysiological activity). In certain embodiments, such neural activity is similar to, or the same as, the wild type neural activity exhibited by the cell in its natural *in vivo* environment.

The term "energetic substrate" as used herein refers to an agent, for example, a sugar, that is a source of energy for a cell for engaging in metabolic processes. In certain embodiments, when a cell is cultured in a medium comprising an energetic substrate, the cell can utilize the energetic substrate for growth and/or maintenance while cultured in the medium.

### II. Media Compositions

In one aspect, the present application provides for a medium that promotes the growth and/or maintenance and/or functional activity of a cell. In certain embodiments, the medium comprises one or more neuroactive inorganic salts and one or more neuroactive amino acids. In certain embodiments, the medium further comprises one or more pH modulating agent, one or more energetic substrate, one or more amino acid, and/or one or more vitamin, and combinations thereof.

In certain embodiments, the ingredients of the medium are present in amounts that promote the growth, maintenance and neural functionality of a neural cell cultured in the medium.

In certain embodiments, the medium does not comprise Hypoxanthine Na, Organosulfur compound (for example, Lipoic Acid), DNA nucleoside (for example, Thymidine), Essential fatty acid (for example, Linoleic Acid), Organic chemical compounds (for example, Putrescine 2HCl), Biotin (B7), and combinations thereof.

In certain embodiments, the medium does not comprise serum.

In certain embodiments, the medium does not comprise one or more ingredients selected from the group consisting of HEPES; Phenol Red; Sodium Pyruvate; BSA, Fatty acid free Fraction V; Catalase; Human recombinant insulin; Human Transferrin; Corticosterone; T3 (triodo-I-thyronine); Linoleic Acid; Linolenic Acid; Ascorbic acid (Vit C); Biotin (B7); DL Alpha Tocopherol Acetate; DL Alpha-Tocopherol; Vitamin A (Retinoic acid); Selenite; D-Galactose; and combinations thereof.

### Neuroactive inorganic salt

In certain embodiments, the medium comprises a neuroactive inorganic salt selected from the group consisting of Sodium Chloride (NaCl), Potassium Chloride (KCl), Calcium Chloride (CaCl₂) (for example, anhydrous CaCl₂), Magnesium Sulfate (MgSO₄) (for example, anhydrous MgSO₄), Magnesium Chloride (MgCl₂) (for example, anhydrous MgCl₂), Ferric Nitrate (Fe(NO₃)₃-9H₂O), Zinc sulfate (ZnSO₄-7H₂O), Cupric sulfate (CuSO₄-5H₂O), Ferric sulfate (FeSO₄-7H₂O), and combinations thereof.

In certain embodiments, the neuroactive inorganic salt is at a concentration of between 0.000001 and 10 mM, or between 0.000005 and 8 mM, or between 0.00001 and 6 mM, or between 0.00005 and 4 mM, or between 0.00005 and 2 mM, or between 0.0001 and 1 mM, or between 0.0005 and 0.5 mM, or between 0.001 and 0.05 mM, or between 0.01 and 0.05 mM.

In certain embodiments, the Sodium Chloride (NaCl) is present at a concentration of between 70 and 150 mM, or between 90 and 130 mM, or between 110 and 125 mM. In certain embodiments, the Sodium Chloride (NaCl) is present at a concentration of 121 mM.

In certain embodiments, the Potassium Chloride (KCl) is present at a concentration of between 0.001 and 10 mM, or between 0.005 and 9 mM, or between 0.01 and 8 mM, or between 0.05 and 7 mM, or between 0.1 and 6 mM, or between 1 and 5 mM, or between 2 and 4.5 mM. In certain embodiments, the Potassium Chloride (KCl) is present at a concentration of less than 5 mM. In certain embodiments, the Potassium Chloride (KCl) is present at a concentration of 4.2 mM.

In certain embodiments, the Calcium Chloride (CaCl₂) (anhydrous) is present at a concentration of between 0.05 and 10 mM, or between 0.1 and 8 mM, or between 0.5 and 6 mM, or between 0.8 and 4 mM, or between 0.8 and 4 mM, or between 0.8 and 2 mM, or between 0.9 and 1.5 mM, or between 0.9 and 1 mM. In certain embodiments, the Calcium Chloride (CaCl₂) (anhydrous) is present at a concentration of 1.1 mM.

In certain embodiments, the Magnesium Sulfate (MgSO₄) (anhydrous) is present at a concentration of between 0.001 and 10 mM, or between 0.005 and 9 mM, or between 0.01 and 8 mM, or between 0.05 and 7 mM, or between 0.1 and 6 mM, or between 0.5 and 5 mM, or between 1 and 4 mM, or between 1.5 and 3. In certain embodiments, the Magnesium Sulfate (MgSO₄) is present at a concentration of less than 2 mM. In certain embodiments, the Magnesium Sulfate (MgSO₄) is present at a concentration of 1 mM.

In certain embodiments, the Magnesium Chloride (MgCl₂) (anhydrous) is present at a concentration of between 0.001 and 10 mM, or between 0.005 and 9 mM, or between 0.01 and 8 mM, or between 0.05 and 7 mM, or between 0.1 and 6 mM, or between 0.5 and 5 mM, or between 1 and 4 mM, or between 1.5 and 3. In certain embodiments, the Magnesium Chloride (MgCl₂) is present at a concentration of less than 2 mM. In certain embodiments, the Magnesium Chloride (MgCl₂) is not present in the medium.

In certain embodiments, the Ferric Nitrate (Fe(NO₃)₃-9H₂O) is present at a concentration of between 0.00001 and 0.005 mM, or between 0.00005 and 0.001 mM, or between 0.0001 and 0.0008 mM, or between 0.0002 and 0.0007 mM, or between 0.0003 and 0.0006 mM, or between 0.0004 and 0.0005 mM. In certain embodiments, the Ferric Nitrate (Fe(NO₃)₃-9H₂O) is present at a concentration of less than 0.0004 mM. In certain embodiments, the Ferric Nitrate (Fe(NO₃)₃-9H₂O) is present at a concentration of 0.000124 mM.

In certain embodiments, the Zinc sulfate (ZnSO₄-7H₂O) is present at a concentration of between 0.00001 and 0.05 mM, or between 0.00005 and 0.005 mM, or between 0.0001 and 0.004 mM, or between 0.0005 and 0.003 mM, or between 0.002 and 0.003 mM. In certain embodiments, the Zinc sulfate (ZnSO₄-7H₂O) is present at a concentration of less than 0.002 mM. In certain embodiments, the Zinc sulfate (ZnSO₄-7H₂O) is present at a concentration of 0.0015 mM.

In certain embodiments, the Cupric sulfate (CuSO₄-5H₂O) is present at a concentration of between 0.0000001 and 0.0005 mM, or between 0.0000005 and 0.00005 mM, or between 0.000001 and 0.00004 mM, or between 0.000005 and 0.00003 mM, or between 0.00001 and 0.00002 mM. In certain embodiments, the Cupric sulfate (CuSO₄-5H₂O) is present at a concentration of less than 0.00001 mM. In certain embodiments, the Cupric sulfate (CuSO₄-5H₂O) is not present in the medium.

In certain embodiments, the Ferric sulfate (FeSO₄-7H₂O) is present at a concentration of between 0.00001 and 0.05 mM, or between 0.00005 and 0.005 mM, or between 0.0001 and 0.004 mM, or between 0.0005 and 0.003 mM, or between 0.001 and 0.004 mM, or between 0.002 and 0.003 mM. In certain embodiments, the Ferric sulfate (FeSO₄-7H₂O) is present at a concentration of less than 0.0015 mM. In certain embodiments, the Ferric sulfate (FeSO₄-7H₂O) is not present in the medium.

### Neuroactive amino acids

In certain embodiments, the medium comprises a neuroactive amino acid selected from the group consisting of Glycine, L-Alanine, L-Aspartic acid, L-Glutamic Acid, L-Serine, and combinations thereof.

In certain embodiments, the neuroactive amino acid is present at a concentration of between 0.0001 and 1 mM, or between 0.0005 and 0.5 mM, or between 0.001 and 0.05 mM, or between 0.005 and 0.01 mM.

In certain embodiments, the Glycine is present at a concentration of between 0.0001 and 0.05 mM. In certain embodiments, the Glycine is present at a concentration of 0.002 mM.

In certain embodiments, the L-Alanine is present at a concentration of between 00001 and 0.05 mM. In certain embodiments, the L-Alanine is present at a concentration of 0.002 mM.

In certain embodiments, the L-Aspartic acid is present at a concentration of between 0.00001 and 0.003 mM. In certain embodiments, the L-Aspartic acid is not present in the medium.

In certain embodiments, the L-Glutamic Acid is present at a concentration of between 0.00001 and 0.02 mM. In certain embodiments, the L-Glutamic Acid is not present in the medium.

In certain embodiments, the L-Serine is present at a concentration of between 0.001 and 0.03 mM. In certain embodiments, the L-Serine is present at a concentration of 0.02 mM.

### pH modulating agent

In certain embodiments, the medium comprises a pH modulating agent wherein the agent is an inorganic salt.

In certain embodiments wherein the pH modulating agent comprises an inorganic salt, the inorganic salt is selected from the group consisting of Sodium Bicarbonate (NaHCO₃), Sodium Phosphate dibasic (Na₂HPO₄) anhydrous, Sodium Phosphate monobasic (NaH₂PO₄-H₂O), and combinations thereof. In certain embodiments, the inorganic salt is at a concentration of between 0.001 and 1 mM.

In certain embodiments, the pH modulating agent comprises Sodium Bicarbonate (NaHCO₃) at a concentration of between 1 and 35mM. In certain embodiments, the NaHCO₃ is at a concentration of 29 mM.

In certain embodiments, the cell medium comprises HEPES at a concentration of between 1 and 10 mM. In certain embodiments, the HEPES is present at a concentration of 5 mM.

The cell medium may comprise phenol red at a concentration of between 0.0001 and 1 mM, or between 0.0005 and 0.5 mM, or between 0.001 and 0.1 mM, or between 0.005 and 0.08, or between 0.01 and 0.07 mM. In certain embodiments, the phenol red is present at a concentration of less than 0.07 mM. In certain embodiments, the phenol red is present at a concentration of 0.0215 mM.

In certain embodiments, the pH of the medium is between 7 and 8, or between 7.1 and 7.8, or between 7.2 and 7.6, or between 7.3 and 7.5. In certain embodiments, the pH of the medium is 7.4.

### Energetic substrate

In certain embodiments, the medium comprises an energetic substrate selected from the group consisting of a sugar (for example, D-glucose (dextrose)), Sodium Pyruvate, and combinations thereof. In certain embodiments, the energy substrate is present at a concentration of between 0.1 and 5 mM. In certain embodiments, the energy substrate is present at a concentration of 2.5 mM or 0.5 mM.

### Growth/maintenance promoting amino acids

In certain embodiments, the medium comprises one or more amino acids. In certain embodiments, the one or more amino acids is selected from the group consisting of L-Alanyl-L-Glutamine, L-Arginine hydrochloride, L-Asparagine-H2O, L-Cysteine hydrochloride-H2O, L-Cystine 2HCl, L-Histidine hydrochloride-H2O, L-Isoleucine, L-Leucine, L-Lysine hydrochloride, L-Methionine, L-Phenylalanine, L-Proline, L-Threonine, L-Tryptophan, L-Tyrosine disodium salt dihydrate, L-Valine, and combinations thereof. In certain embodiments, each amino acid is present at a concentration of between 0.001 and 1 mM.

In certain embodiments, the one or more amino acids is selected from the group consisting of L-Alanyl-L-Glutamine at a concentration of 0.5 mM, L-Arginine hydrochloride at a concentration of 0.5 mM, L-Asparagine-H2O at a concentration of 0.05 mM, L-Cysteine hydrochloride-H2O at a concentration of 0.15 mM, L-Histidine hydrochloride-H2O at a concentration of 0.2 mM, L-Isoleucine at a concentration of 0.8 mM, L-Leucine at a concentration of 0.8 mM, L-Lysine hydrochloride at a concentration of 0.8 mM, L-Methionine at a concentration of 0.2 mM, L-Phenylalanine at a concentration of 0.4 mM, L-Proline at a concentration of 0.1 mM, L-Threonine at a concentration of 0.7 mM, L-Tryptophan at a concentration of 0.07 mM, L-Tyrosine disodium salt dihydrate at a concentration of 0.4 mM, L-Valine at a concentration of 0.7 mM, and combinations thereof.

In certain embodiments, the medium does not comprise L-Cystine 2HCl.

### Growth/maintenance promoting vitamins

In certain embodiments, the medium comprises one or more vitamins. In certain embodiments, the one or more vitamins is selected from the group consisting of Choline chloride, D-Calcium pantothenate (B5), Folic Acid (B9), i-Inositol, Niacinamide (B3), Pyridoxine hydrochloride, Thiamine hydrochloride, Vitamin B12 (cyanocobalamin), Riboflavin (B2), and combinations thereof. In certain embodiments, each vitamin is present at a concentration of between 0.00001 and 1 mM, or between 0.00005 and 0.5 mM, or between 0.0001 and 0.9 mM, or between 0.0005 and 0.8 mM, or between 0.001 and 0.7 mM, or between 0.005 and 0.6 mM, or between 0.01 and 0.5 mM, or between 0.05 and 0.1 mM.

In certain embodiments, the one or more vitamins is selected from the group consisting of Choline chloride at a concentration of 0.07 mM, D-Calcium pantothenate (B5) at a concentration of 0.006 mM, Folic Acid (B9) at a concentration of 0.006 mM, i-Inositol at a concentration of 0.07 mM, Niacinamide (B3) at a concentration of 0.02 mM, Pyridoxine hydrochloride at a concentration of 0.010 mM, Thiamine hydrochloride at a concentration of 0.007 mM, Vitamin B12 (cyanocobalamin) at a concentration of 0.0006 mM, Riboflavin (B2) at a concentration of 0.0006 mM, and combinations thereof.

### Supplemental agents

In certain embodiments, the medium further comprises a supplemental agent selected from the group consisting of protein (for example, Laminin; BSA, Fatty acid free Fraction V; Catalase; insulin; Human recombinant insulin; Insulin Recombinant Full Chain; Transferrin; Human Transferrin; Human Transferrin (Holo); Superoxide Dismutase), neurotrophic factor (for example, Human Brain Derived Neurotrophic Factor (BDNF); Glia neurotrophic factor (GDNF)), hormone, steroid (for example, Corticosterone, Progesterone), hormone thyroid (for example, T3 (triodo-I-thyronine)), fatty acid, essential fatty acid (for example, Linoleic Acid, Linolenic Acid), lipid (for example, Cholesterol), Vitamin (for example, Ascorbic acid (Vit C), Biotin (B7), DL Alpha Tocopherol Acetate, DL Alpha-Tocopherol, Vitamin A (Retinoic acid)), Sulfate mineral (for example, selenite), Organic chemical compound (for example, Putrescine 2HCl), Monosacharide (for example, D-Galactose), Nucleotide (for example, Dibutyril cAMP sodium salt), and combinations thereof.

In certain embodiments each supplemental agent is present in the medium at a concentration of between 0.01 and 50 µg/mL, or between 1 and 40 µg/mL, or between 5 and 30 µg/mL, or between 10 and 20 µg/mL.

In certain embodiments each supplemental agent is present in the medium at a concentration of between 0.01 and 50 mg/mL, or between 1 and 40 mg/mL, or between 5 and 30 mg/mL, or between 10 and 20 mg/mL.

In other embodiments, each supplemental agent is present in the medium at a concentration of between 0.000001 and 1 mM, or between 0.00001 and 0.5 mM, or between 0.0001 and 0.1 mM, or between 0.001 and 0.01 mM.

### Additional medium formulations

The medium may comprise L-alanine at a concentration below 0.05 mM, glycine at a concentration below 0.25 mM, L-serine at a concentration below 0.25 mM, L-proline at a concentration below 0.15 mM, L-arginine hydrochloride at a concentration below 0.60 mM, L- alanyl-L-glutamine at a concentration below 2.5 mM, magnesium sulfate at a concentration above 0.41 mM, calcium chloride at a concentration above 1.05 mM, potassium chloride at a concentration above 4.16 mM, sodium chloride at a concentration above 120.7 mM, D-glucose at a concentration below 17.5 mM or HEPES at a concentration of 5 mM. In some embodiments, the medium does not include L-aspartic acid, L-glutamic acid, L-cystine 2HCl, CuSO₄-5H₂O, FeSO₄-7H₂O, anhydrous magnesium chloride, linoleic acid, putrescine 2HCl, lipoic acid, thymidine, hypoxanthine Na, or biotin.

In some embodiments, the L-alanine is present between 0.001 mM and 0.05 mM. In other embodiments, the L-alanine is present at 0.002 mM. In some embodiments, the glycine is present between 0.001 mM and 0.005 mM. In other embodiments, the glycine is present at 0.002 mM. In some embodiments, the L-serine is present between 0.001 mM and 0.005 mM. In some embodiments, the L-serine is present at 0.002 mM. In other embodiments, the L-proline is present between 0.01 mM and 0.1 mM. In some embodiments, the L-proline is present at 0.06 mM. In other embodiments, the L-arginine hydrochloride is present between 0.01 mM and 0.5 mM. In some embodiments, the L-arginine hydrochloride is present at 0.3 mM. In some embodiments, the L-alanyl-L-glutamine is present between 0.1 mM and 1 mM. In other embodiments, the L-alanyl-L-glutamine is present at 0.5 mM.

In some embodiments, the magnesium sulfate is present between 0.5 mM and 5 mM. In other embodiments, the magnesium sulfate is present at 1 mM. In some embodiments, the calcium chloride is present between 1.1 mM and 2 mM. In some embodiments, the calcium chloride is present at 1.1 mM. In other embodiments, the potassium chloride is present between 4.18 mM and 4.5 mM. In some embodiments, the potassium chloride is present at 4.2 mM. In some embodiments, the sodium chloride is present between 121 mM and 125 mM. In other embodiments, the sodium chloride is present at 121 mM. In some embodiments, the D-glucose is present between 1 mM and 10 mM. In other embodiments, the D-glucose is present at 2.5 mM.

In some embodiments, the medium includes L-alanine at a concentration of 0.002 mM, glycine at a concentration of 0.002 mM, L-serine at a concentration of 0.002 mM, L-proline at a concentration of 0.06 mM, L-arginine hydrochloride at a concentration of 0.3 mM and L-alanyl-L-glutamine at a concentration of 0.5 mM.

In some embodiments, the medium includes magnesium sulfate at a concentration of 1 mM, calcium chloride at a concentration of 1.1 mM, potassium chloride at a concentration of 4.2 mM and sodium chloride at a concentration of 121 mM.

In some embodiments, the medium includes L-alanine at a concentration below 0.05 mM, glycine at a concentration below 0.25 mM, L-serine at a concentration below 0.25 mM, L-proline at a concentration below 0.15 mM, L-arginine hydrochloride at a concentration below 0.60 mM, L-alanyl-L-glutamine at a concentration below 2.5 mM, magnesium sulfate at a concentration above 0.41 mM, calcium chloride at a concentration above 1.05 mM, potassium chloride at a concentration above 4.16 mM, sodium chloride at a concentration above 120.7 mM, D-glucose at a concentration below 17.5 mM and HEPES at a concentration of 5 mM.

### Osmolarity

In some embodiments, the medium has an osmolarity below 315 mOsmol/L. In other embodiments, the osmolarity is between 280 and 330 mOsmol/L, or between 300 and 320 mOsmol/L. In certain embodiments, the osmolarity is between 300 mOsmol/L and 310 mOsmol/L. In some embodiments, the osmolarity is 310 mOsmol/L.

### III. Methods of Culturing and Cellular Compositions

In another aspect, a method of culturing a neuronal cell is provided. The method includes, contacting a neuronal cell with a medium as provided herein including embodiments thereof and allowing the neuronal cell to grow, thereby culturing a neuronal cell. In some embodiments, the neuronal cell is a primary neuronal cell. In other embodiments, the neuronal cell is an iPSC-derived neuronal cell.

In another aspect, a composition comprising a neuronal cell cultured in a medium as provided herein including embodiments thereof is provided. In certain embodiments, the medium comprises one or more ingredient that is sensitive to energetic sources, such as, for example, light and/or electricity. In certain embodiments, contacting the ingredient with energy, such as light or electricity, results in an increase in activity of a cell cultured in the medium, for example, a neural cell. In certain embodiments, continued application of the energy to the medium may produce an excitotoxic effect in the cell cultured in the medium. In certain embodiments, the ingredient that is sensitive to energy is selected from the group consisting of Riboflavin (B2), HEPES, and combinations thereof. In certain embodiments, the agent is present at a concentration of between 0.0001 and 0.0006 mM. In certain embodiments, the agent is present at a concentration of between 1 and 10 mM.

### Examples

### Neurophysiological medium supporting the synaptic activity and function of human brain cells in vitro

### Summary

Induced pluripotent stem cell (iPSC) technologies offer access to live human neurons derived from patients and a new alternative to model neurological and psychiatric disorders *in vitro.* Effective models of neuronal circuits require physiological conditions that sustain neuronal functions. Therefore, Applicant specifically examined the neuronal activity in different media currently used to culture neurons. Neurons from rodents and humans are routinely grown *in vitro* in basal media with a variety of supplements. It was found that standard basal media (e.g. DMEM, Neurobasal) and serum strongly impair fundamental neuronal functions, including action potential firing and synaptic communication. To overcome these limitations and better reproduce *in vitro* the physiological conditions of the human brain, a new basal medium (BrainPhys) was designed, and it was combined with chemically-defined supplements and thoroughly tested it on a variety of human neuronal cultures. This new medium optimally supports the type of electrophysiological activity typically observed *in vivo,* but in addition it also sustains long-term survival *in vitro.* BrainPhys was essentially designed to culture functional and mature human neurons obtained from patients or healthy subjects but can also be used on rodent neurons. Overall, it provides more realistic and functional *in vitro* conditions to model human neurological diseases.

The present example describes the following:
∘ Classic basal medium (DMEM and Neurobasal) and serum strongly impair fundamental functions of human neurons *in vitro* (action potentials and excitatory/inhibitory synaptic activity)
∘ BrainPhys-based medium without serum supports optimal neuronal functions (action potentials/ synaptic communication) while also sustaining neuronal maturation and brain cell survival *in vitro*
∘ BrainPhys-based medium increases the proportions of mature neurons with active and functional synaptic connectivity
∘ BrainPhys with serum-free supplements can sustain stable and healthy neuronal activity over several weeks and reduce fluctuations linked with feeding cycles observed in DMEM-based medium
∘ BrainPhys composition better mimics CSF neurophysiological conditions and provides more realistic conditions to *in vitro* models of human neurological and psychiatric diseases to increase the chances of translational success

### Results

To assess the neural network activity of neurons in culture, human neural progenitor cells (NPCs) were used that were derived from iPSCs or embryonic stem cells (ESCs). The human NPCs were differentiated into neurons on glass cover slips with various basal media that were supplemented with N2, B27, BDNF, GDNF, Ascorbic acid, cAMP and Laminin for two to eight weeks. The coverslips were transferred with the neurons in a perfusion chamber to acutely examine the spontaneous calcium activity or electrophysiological properties of the cells in various extracellular solutions.

### DMEM/F12 based medium impairs calcium activity, spontaneous action potential firing, and synaptic activity in human neurons

The calcium activity of human neurons was first imaged in the DMEM-based media, in which they were cultured for several weeks. Very few active human neurons were found, but when the same fields of view were imaged in ACSF, many more cells became spontaneously active. The composition of ACSF solution may slightly vary between laboratories - for example, calcium concentration is often higher than physiological levels to artificially increase synaptic release - which can potentially increase network activity. To clarify this issue, The inorganic salt concentration, the pH, and osmolarity of our ACSF was matched to those in DMEM and those calcium-imaging experiments were repeated. Despite those precautions, it was confirmed that the number of active cells was significantly lower in DMEM than in ACSF (**Fig 1a**).

Patch-clamping methods were then used to determine how DMEM reduced the overall spontaneous activity of human neurons *in vitro.* It was found that DMEM consistently depolarized the resting potential of neurons (n = 22/22 cells, by 23±3 mV). Spontaneously active neurons in ACSF were then examined, and it was found that, on rare occasions, depolarizations induced by DMEM increased the firing frequency without saturation (n = 2/14, **Fig. 9a**), but in most cases it saturated and silenced the firing of the cells (n = 12/14, **Fig 1b****,** **Fig. 9b**).

To investigate the influence of DMEM on synaptic function, voltage clamp experiments were performed at the reversal potential of Cl⁻ (-70 mV) or Na+ (0 mV), and it was found that both spontaneous AMPA and GABA synaptic events, which could be recorded in ACSF, completely disappeared in DMEM (**Fig. 1b**). This effect was reversible and occurred in every tested neuron (n = 6/6 cells).

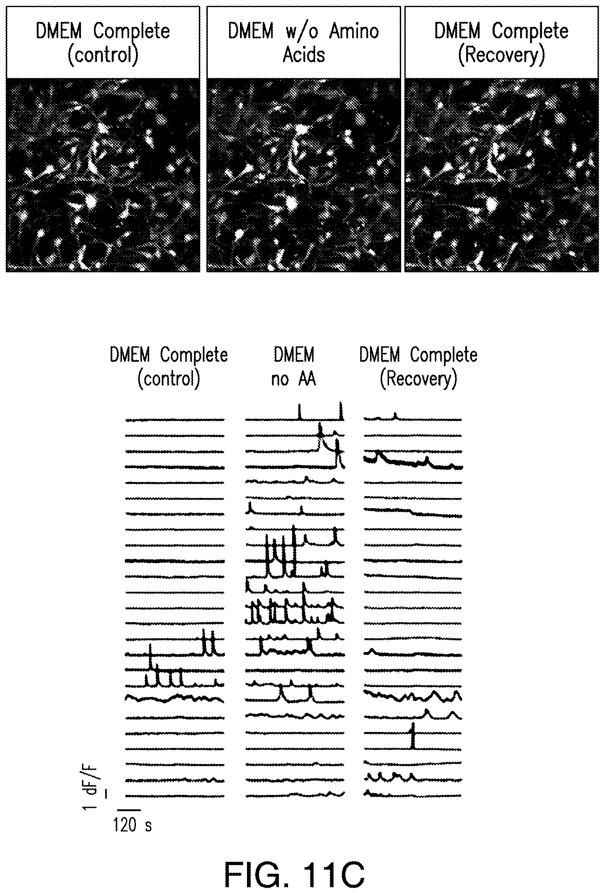

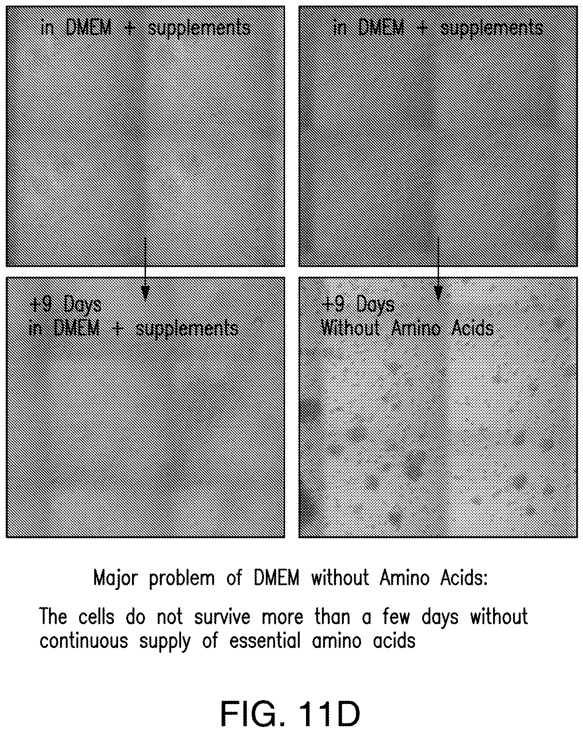

At the same time, it was also observed that perfusion of DMEM consistently induced large depolarizing Na+ and Cl- influx into the cells (e.g., **Fig. 10**)**.** Therefore, it was suspected that components present in the DMEM but not in ACSF may activate Na+ and Cl- channels on the neurons. Therefore either all the vitamins, all the amino acids or all the extra components of DMEM were removed and it was found that by removing the bulk of the amino acids, the DMEM-induced depolarizations that caused the impairment of action potentials and synaptic activity in DMEM could be avoided. However, this solution failed to sustain the development and survival of the neurons for more than a week (**Fig. 11**). From these experiments, it was clear that neuroactive amino acids in DMEM impaired basic neuronal functions, including action potential propagation and synaptic communication.

### Neurobasal based medium reduces synaptic communication and action potential firing

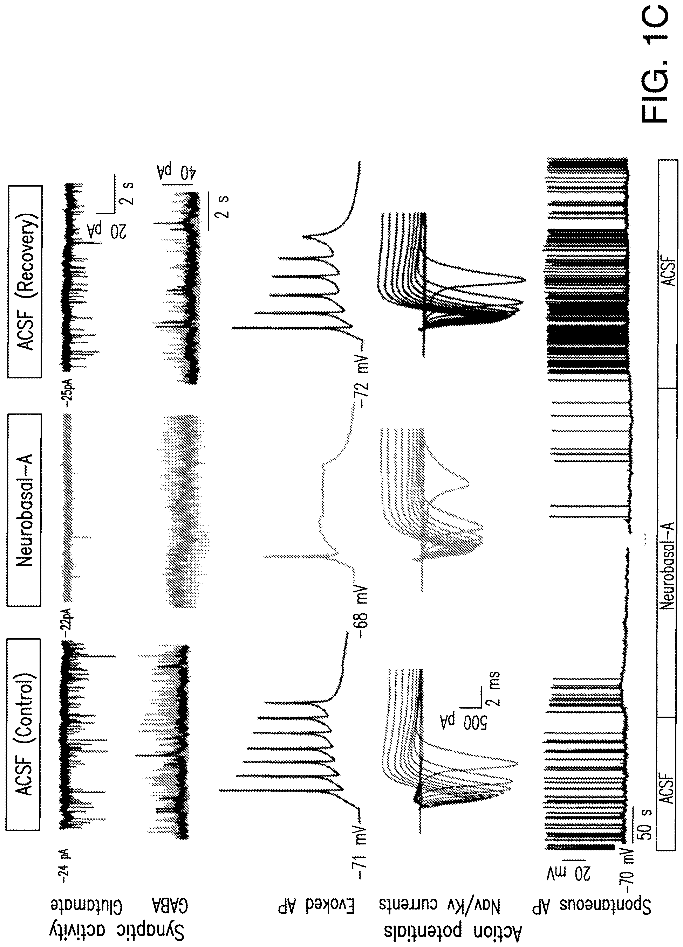

DMEM has been previously modified to optimize the survival of rat primary neurons in culture (Brewer *et al.,* 1993). In this modified version, called Neurobasal medium, the developers essentially removed some excitatory amino acids and ferrous sulphate, and reduced the osmolarity; they found that Neurobasal improved rat neurons survival *in vitro* in comparison to DMEM (Brewer *et al.,* 1993). Although cell survival is a critical parameter of cell culture, fundamental electrophysiological properties were not tested in Neurobasal. Therefore, human iPSC- and ESC-derived neurons (two to eight weeks in neural maturation media) were used to examine the effects of Neurobasal on neuronal functions. Unlike DMEM, Neurobasal did not depolarize the resting membrane potential and occasionally at least some excitatory synaptic events could be observed. Nevertheless, Neurobasal strongly reduced or abolished the spontaneous excitatory synaptic activity observed in ACSF (n = 5/5 mature neurons, ACSF control: 21.3±12.5 Hz, Neurobasal-A: 0.44±0.17 Hz, ACSF recovery: 15.1±10.5 Hz; **Fig 1c**). The concentrations of inorganic salts and the osmolarity of Neurobasal are very different to what the neurons are naturally exposed to in the brain (Davson and Segal, 1996). As a likely consequence, it was found that Neurobasal significantly reduced the voltage-dependent sodium currents and rapidly inactivating potassium currents and impaired the amplitude and frequency of evoked and spontaneous action potentials (n = 8/8 cells; **Fig 1c**). Attemps to increase the concentration of NaCl in Neurobasal were first tried, however despite some slight improvements in the amplitude of voltage dependent sodium currents, this modification was not sufficient to optimize the electrical and synaptic activity of the human neurons. It was therefore suspected that like in DMEM, several neuroactive components in Neurobasal were preventing neurophysiological activity. Since neither DMEM nor Neurobasal were capable of sustaining essential electrophysiological neuronal properties such as action potential firing and synaptic function, a new basal medium was designed that is better adapted to neuronal function.

### New BrainPhys basal medium supports functional action potential firing

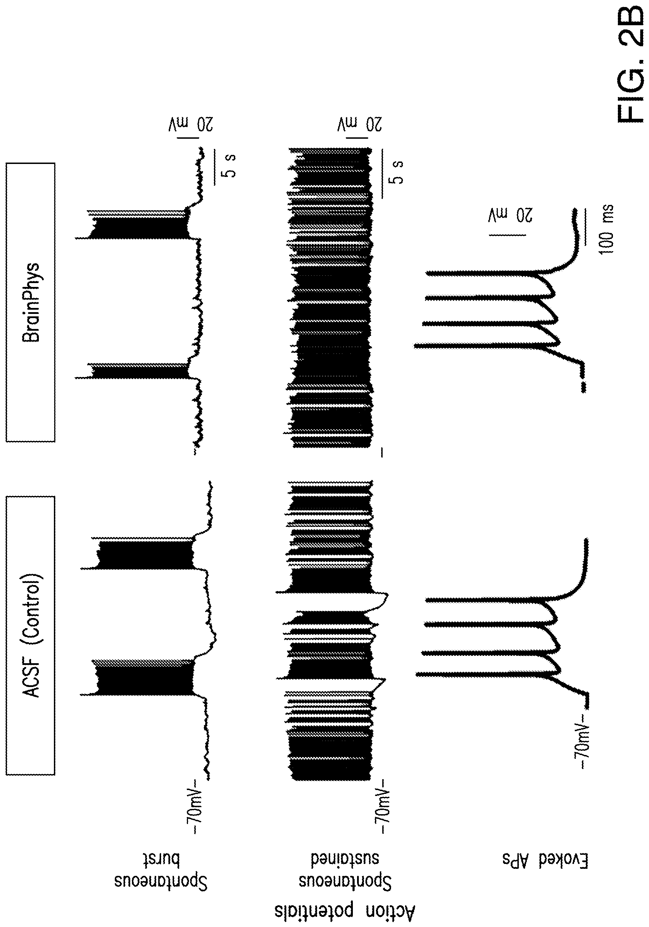

Neuronal function is fundamentally based on the generation and propagation of action potentials. Voltage-dependent sodium (Nav) and potassium (Kv) channels are crucial to achieving high firing rates of action potentials. Action potentials are reflecting the sequential activation Nav and Kv channels that trigger large outflux of sodium and influx of potassium currents largely depending on the ionic gradients. The first improvement of BrainPhys was therefore to adjust the concentrations of inorganic salts close to neurophysiological levels (e.g. Na+, Cl-, K+, Ca2+). Tests in voltage-clamp showed that the amplitude of Nav and Kv currents in ACSF and BrainPhys were not different (**Fig. 2a**). Consequently, spontaneous and evoked action potentials in single neurons tested in BrainPhys were also considerably improved, up to similar levels measured in ACSF (n = 3 cells; **Fig 2b**). Likewise, the spontaneous activity measured at the network level with calcium imaging in ACSF and BrainPhys was comparable.

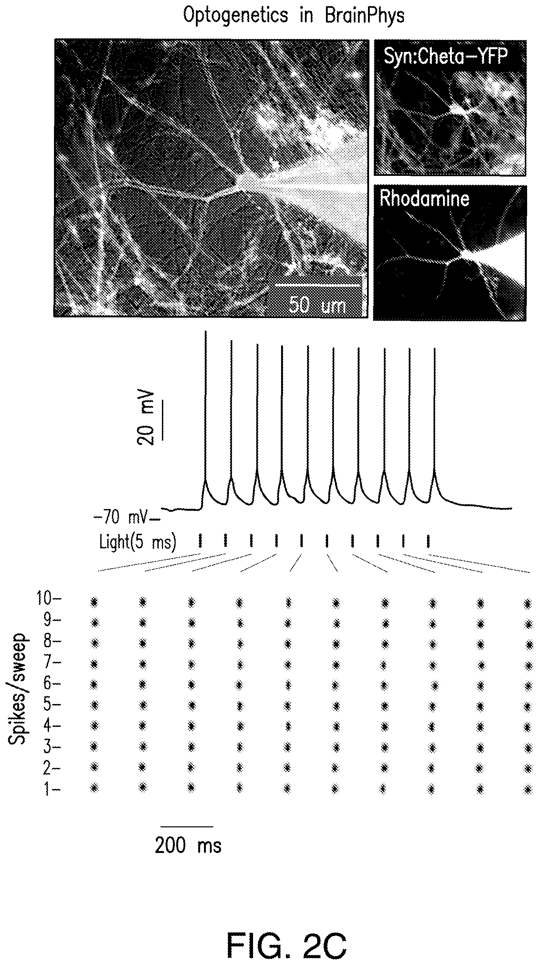

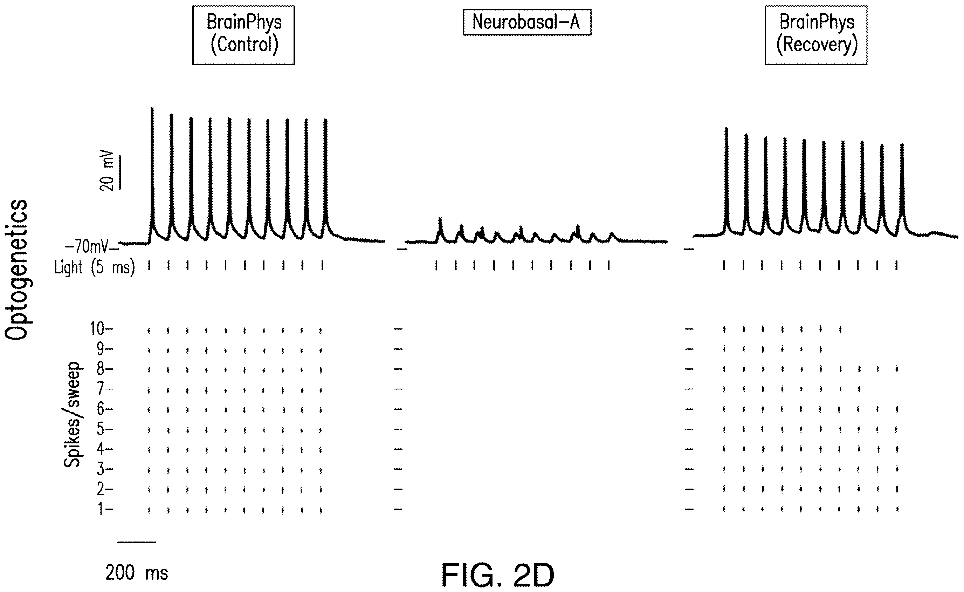

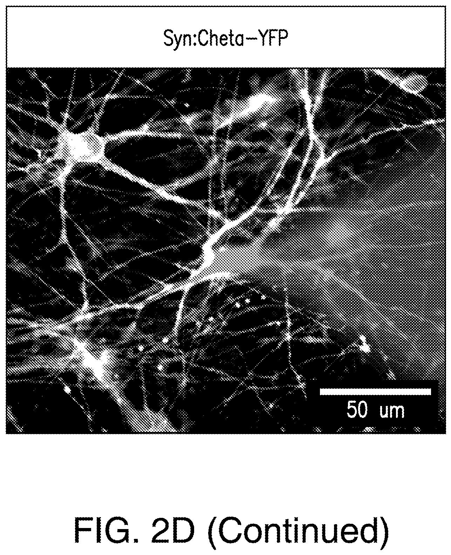

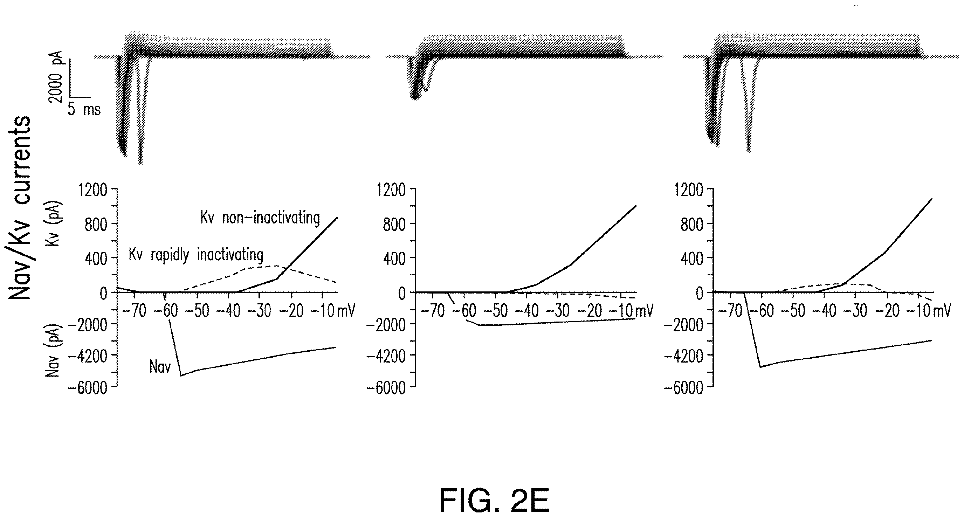

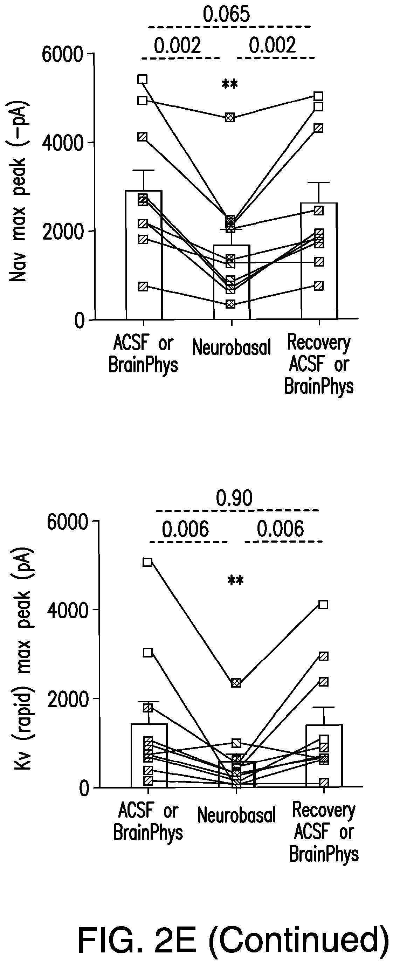

In addition, it was shown that optogenes such as Chanel Rhodopsin (ChR2) or Cheta can be efficiently expressed in human neurons and allowed us to remotely control the firing activity of the neurons with brief flashes of light in BrainPhys (**Fig. 2c**). In sharp contrast LED stimulations of neurons that successfully evoked responses in BrainPhys, failed to evoke clear action potentials in either Neurobasal or DMEM (**Fig. 2d**). Consistent with the overall improvement in firing activity, the amplitudes of Nav and rapidly inactivating Kv currents were systematically larger when recorded in BrainPhys or ACSF compared to Neurobasal (**Fig 2e**).

### New BrainPhys supports functional excitatory and inhibitory synaptic activity

Integrated neural network activity and ultimately brain functions are achieved by synaptic communication between neurons. Therefore, we designed BrainPhys to sustain both excitatory and inhibitory synaptic function. The summation of excitatory and inhibitory synaptic neurotransmitters determines the probability of neurons to fire action potentials. Thus simply by supporting optimal action potential firing, BrainPhys indirectly acts to promote synaptic activity. In addition, specific ions such as Calcium play critical roles in the synapse to trigger fast vesicular release. Aiming to set *in vitro* conditions as realistic as possible, we adjusted calcium levels in BrainPhys close to those reported in the human cerebrospinal fluid in vivo (Ca2+ ∼1.1 mM). However, based on voltage clamp experiments showing strong Na+ and/or Cl-currents upon perfusion of DMEM or Neurobasal, we strongly suspected that synaptic function was also impaired by the presence of neuroactive components.

The main excitatory synaptic connections in the brain are mediated by glutamate while most synaptic inhibition is mediated by GABA. Therefore, the neuroactive elements in classic media that could directly influence glutamatergic and gabaergic synaptic activity were excluded or reduced. To control that those modifications improved synaptic functions, the levels of spontaneous glutamatergic and gabaergic synaptic activity, mediated by AMPA or GABAa receptors were tested in voltage-clamp (as shown by reversible blockades with their respective antagonists NBQX or SR95531). Although spontaneous activity may be variable between cells, a paired analysis clearly showed that the levels of spontaneous AMPA synaptic activity in BrainPhys were not significantly different to those in ACSF (n = 5 cells, ACSF: 11.0±10 Hz, BrainPhys: 12.8±10 Hz), and therefore largely improved compared to DMEM or even Neurobasal (**Fig. 2f**).

It was systematically found that both Neurobasal and DMEM completely blocked synaptic inhibitory phasic activity (n= 4/4 cells in Neurobasal; **Fig 1c**). Since the blockade of GABA synaptic activity was accompanied by large influx of chloride currents (**Fig. 14**), it was suspected that the blockade was due to the presence of neuroactive components saturating chloride inotropic channels.

Following this hypothesis, it was demonstrated that reducing the concentration of specific neuroactive components acting of chloride channels dramatically improved GABAergic synaptic activity. As a result of those modifications spontaneous GABA synaptic activity was proven functional in BrainPhys (n = 7 cells; **Fig 2f**) and comparable to levels of activity in ACSF (n = 3 cells).

In summary, these results show that unphysiological concentration of neuroactive components present in DMEM and Neurobasal strongly impaired excitatory and inhibitory synaptic activity. This critical caveat was therefore fixed through the formulation of BrainPhys and it was shown that synaptic communication is active and functional in BrainPhys. Taken all together, the improvements made in BrainPhys to support physiological resting membrane potential, action potential firing and synaptic function were also reflected by the presence of healthy calcium network activity comparable to ACSF.

### BrainPhys better mimic the human brain physiological environment

The primary objective was to design a medium that better supports neuronal functions and activity *in vitro.* But it was also an objective to design a medium that would better mimic the basic conditions in the human brain. Those improvements are not only important for studies directly focusing on neuronal activity but are also potentially critical for disease modelling studies, as more realistic experimental models will increase translational success for patients.

To reach that goal, it has been reported to be essential to provide similar energetic levels that are normally rigorously maintained in the healthy human brain (Zilberter *et al.,* 2010). This is particularly important since several neurological disorders have been related to mitochondria dysfunctions (Knott *et al.,* 2008; Cooper *et al.,* 2012; Itoh *et al.,* 2012). Surprisingly though, the glucose levels in DMEM and Neurobasal are at least two to five times higher than those in the brain of hyperglycemic patients (Gruetter *et al.,* 1992). The energetic components in BrainPhys were therefore balanced to provide glycemic levels similar to those reported for the brains of healthy patients (∼2.5 mM).

Furthermore, while adjusting the concentration of inorganic salts to physiological levels, and avoiding the saturation of neuroactive components, the osmolarity was also set to be close to that of typical human cerebrospinal fluid (∼305 mOsmol/L). In contrast, the osmolarity of Neurobasal is ∼30% lower than neurophysiological levels (∼220 or 250 mOsmol/L for Neurobasal-A).

Although it has been shown that those modifications accounted for better neuronal function and were closer to neurophysiological conditions, it was crucial to make those improvements while maintaining conditions that can also sustain brain cell survival for several weeks or perhaps months in an artificial *in vitro* setting. The influence of maturing human neurons in BrainPhys were thus tested.

### Characterisation of healthy active neural network which matured for several weeks in BrainPhys-based serum-free media without feeder layer

Different types of neurons can be used to study human neurons *in vitro* (e.g. cortical, dopaminergic), and the methodological variations to obtain those neurons are countless. However, after a specific type of neuronal progenitor is obtained, usually, the cells are matured in a basal medium with supplements. Typically, DMEM, Neurobasal or a mixture of both is picked as basal medium. The supplements added to the basal media can be tailored depending on the need or preference of the experimenter. We first tested that acutely adding a complete set of commonly used supplements (N2, B27, Retinoic acid, BDNF, GDNF, ascorbic acid, cAMP, laminin, and cholesterol) to BrainPhys basal medium did not affect the firing rate or excitatory/inhibitory synaptic activity (n = 6 cells, **Fig. 12**). However we found that serum (e.g. fetal bovine serum, FBS, or chemically defined serum), which is occasionally added to neuronal media strongly impaired the neuronal activity (**Fig 6a**). Therefore, all the experiments were performed in serum-free media. In addition, serum can introduce variability from batch to batch. Likewise, antibiotics were not used, experiments was performed in sterile conditions and the supernatant was tested weekly for mycoplasma.

It was tested that BrainPhys-based neuronal media could be proficient to use with a large variety of neurons, including for example human ES and IPS cells-derived neurons, iNs directly reprogrammed from human fibroblast, primary neurons and even ex-vivo brain slices. To test the cell physiological properties with patch-clamping, calcium imaging and confocal IHC after maturing for several weeks in BrainPhys-based medium with supplements (N2, B27, Retinoic acid, BDNF, GDNF, ascorbic acid, cAMP, Laminin), almost all the neurons were cultured on transferable coated glass coverslips, but BrainPhys can also be used to feed neurons plated on plastic, which may improve cell attachment. Adding a feeder layer of glia may also improve the long-term attachment of the neurons and provide other benefits, but it was opted to omit feeder layers in most of the tests since it was noticed that the glial progenitors often turn into neurons in neural maturation media. This may be a concern for some disease modelling studies as it may contaminate the neuronal samples with cells from another patient or even another species if rodent astrocytes are used.

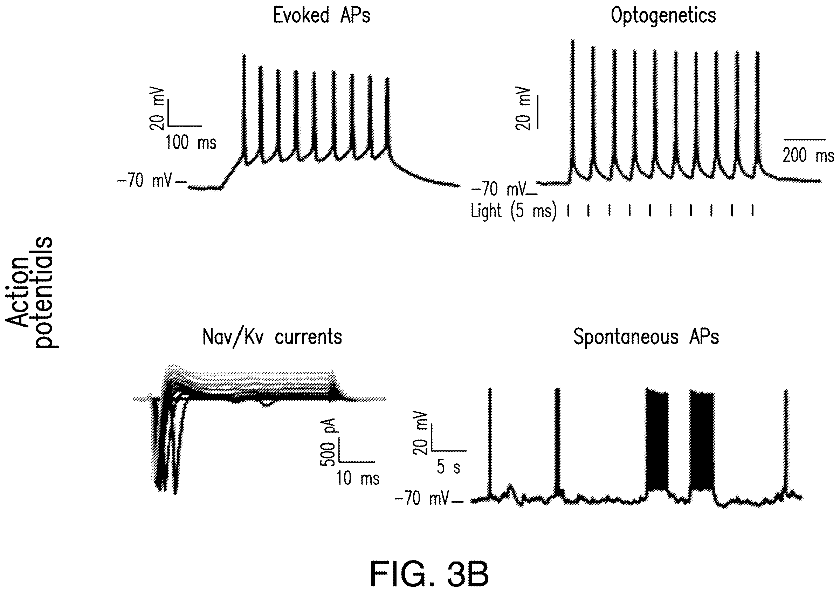

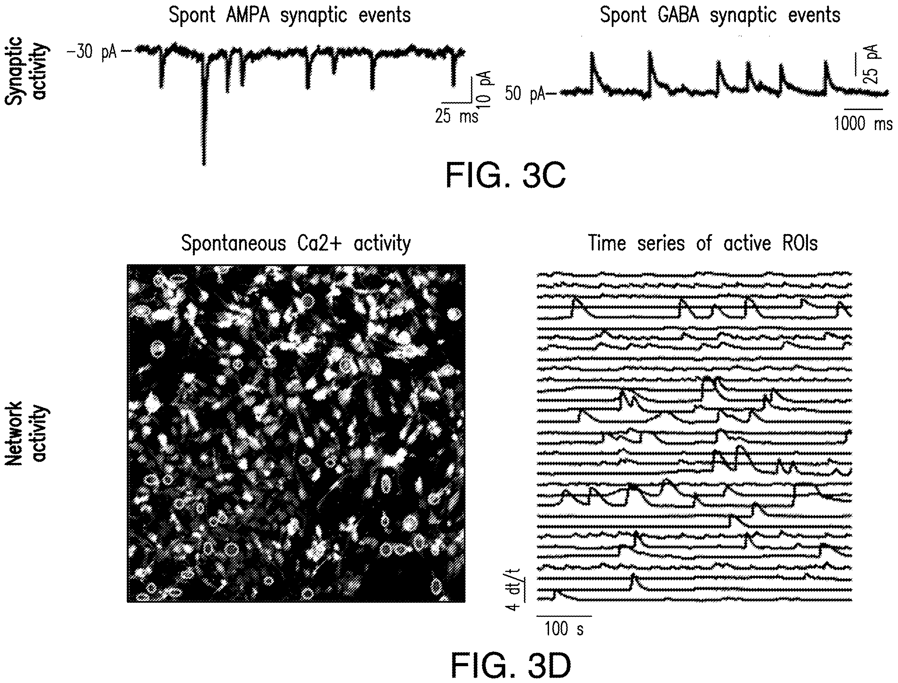

It was tested extensively that BrainPhys (with supplements) could be used to differentiate human iPSC- and ESC-derived NPCs into neuronal networks on glass coverslips. It was found that human NPCs plated in BrainPhys differentiated effectively into mature and synaptically active neurons within 3 to 5 weeks (**Fig 3**), although this may vary depending on the cell line and the reprogramming protocol. The neurons survived on glass coverslips for several weeks or even several months for the best cell lines (tested up to 4 months). After ∼4 weeks of maturation the culture formed dense neuronal networks and nicely stained for dendritic/axonal markers (MAP2 and TUJ1), synaptic markers (Synapsin) and neuronal markers (TH, GABA, VGlut) (**Fig 3a**). Consistent with previous observations, human NPCs differentiated not only into neurons but also into a population of astrocytes staining positively for GFAP (**Fig 7**).

Patch-clamp recordings in BrainPhys (with supplements) revealed that iPSC or ESC-derived neurons maturing in BrainPhys (with supplements) for several weeks were firing healthy trains of action potentials (**Fig 3b**) and received both spontaneous AMPA and GABA synaptic activity (**Fig 3c**). Calcium imaging experiments also demonstrated that BrainPhys-based neural cultures generated functionally active neural networks (**Fig 3d**).

### BrainPhys improved the functional maturation of human iPSC- and ESC-derived NPCs

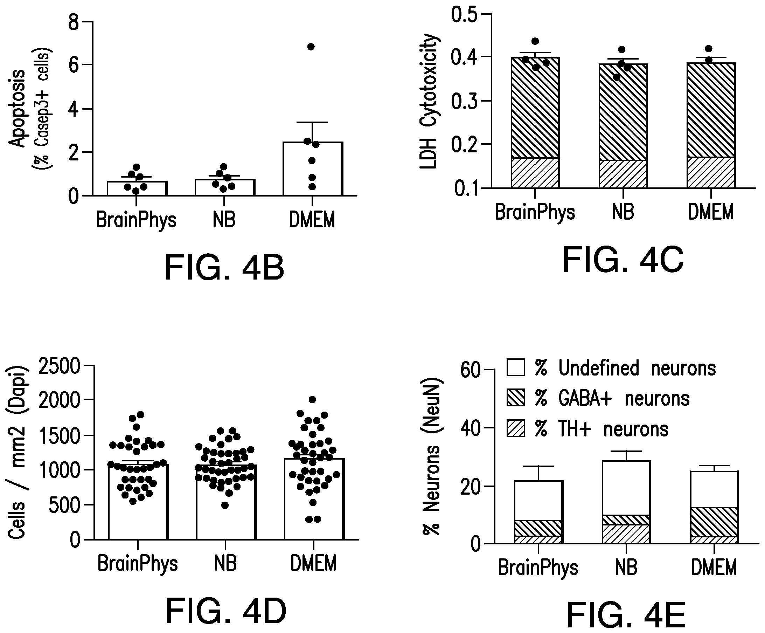

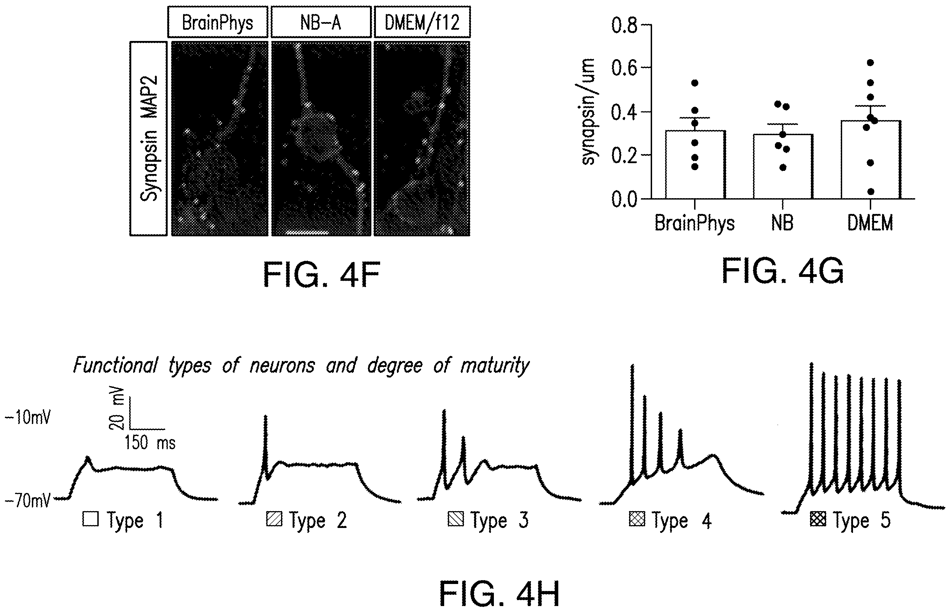

DMEM and Neurobasal medium were specifically optimized to promote the survival of cells in vitro. To test the viability of the cells in different basal media, DMEM, Neurobasal and BrainPhys (all with the same set of serum free supplements), matched experiments were performed using the same cell lines plated at similar densities at the same time, but fed for several weeks with different basal media. It was found that after 1 month in either medium the proportion of apoptotic cells (active caspase 3), the overall cell density (DAPI) or the concentration of LDH released in the supernatant by dying cells did not change significantly between the three groups (**Fig 4a****-d**). Significant differences were not found in the proportion of GABA neurons, dopaminergic neurons (TH) or NeuN positive neurons (**Fig 4a****,e**). Similarly, after 4 weeks maturation in either based media, obvious differences in the density of synaptic puncta on the proximal dendrites were not observed (**Fig 4f****-g**).

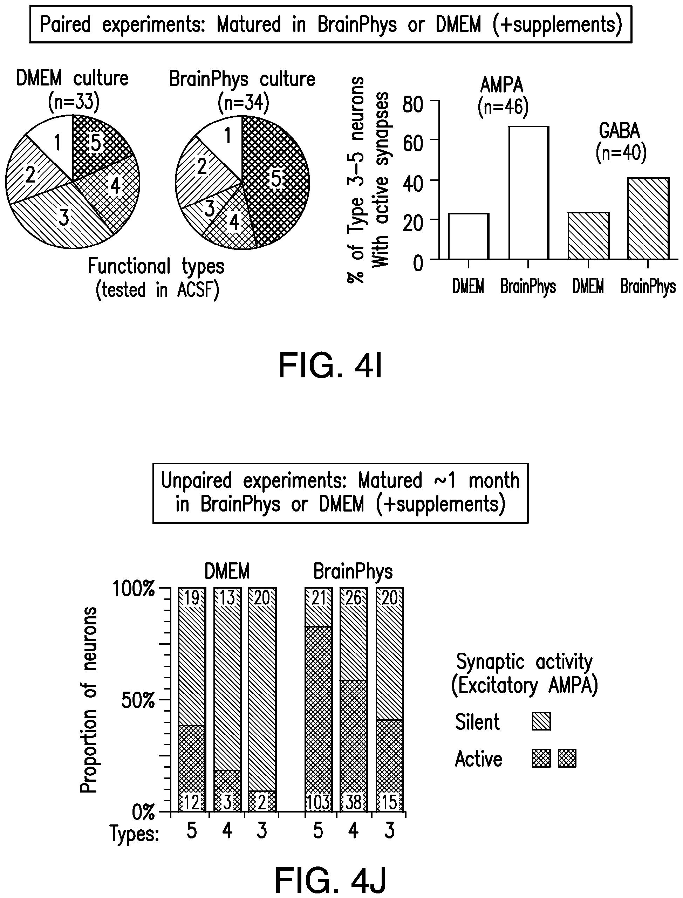

Since BrainPhys did not appear to affect the survival or the fate of the cells, it was then asked whether growing cells for 2 to 6 weeks in active BrainPhys-based medium may improve the functional maturity of the neurons. To assess the degree of functional maturity, a homogeneous sample of 65 synapsin-GFP-positive neurons were first randomly patched in cultures growing side by side in DMEM or BrainPhys (with supplements) (**Fig 4**). To avoid any possible bias due to tissue culture variability, the same NPCs were plated down, at the same density (∼2x10⁵ cells per well; 190 mm²), in the same plate, at the same time, and the few coverslips in each groups with lower cell density due to detachment of the cells from the coverslip and/or large clumps of cells were discarded. The neurons growing in either BrainPhys or DMEM (with supplements) that were selected for patch-clamping had on average the same number of primary neurites (BrainPhys: 3.5+0.2 um, DMEM: 3.5±1.2 um). The patching of neurons that were cultured in BrainPhys or DMEM were blindly alternated; therefore, the age of the neurons was virtually identical in both groups (average 28 vs 29 days, respectively, ranging from 17 to 45 days,) (**Table 4**). The patched neurons were differentiated in either DMEM- or BrainPhys-based media, but for these experiments their functional properties in ACSF were acutely assessed. In these conditions there was reasonable confidence that the only difference between the coverslips selected for analysis (n = 22) was the medium used for feeding. Despite efforts to homogenize the sampling of recorded neurons, more mature neurons were obtained in the BrainPhys cultures. To quantify this result, different functional types of neurons were defined based on their firing pattern in response to 500 ms depolarizing steps of currents (**Fig 4h****,** see **methods** for classification details). After two to six weeks of maturation in respective media and electrophysiological testing in ACSF, half (47%) of the neurons differentiated in BrainPhys-based media were classified as functional Type 5 neurons, which was more than twice more Type 5 neurons than in the matched DMEM-based cultures **(****Fig 4i****).** It was also consistently measured that Nav and rapidly inactivating Kv currents were significantly higher in neurons from BrainPhys-based cultures, but their voltage activation threshold was similar (**Table 4**). In addition, the membrane resistance of BrainPhys-grown neurons was significantly lower and the capacitance significantly higher than in DMEM-grown neurons, characteristics that also typically correlate with more mature-like properties (**Table 4**).

Despite the lack of obvious increase in the number of synaptic puncta measured by IHC, it was found that the number of functionally active synapses was largely increased in neurons differentiated in BrainPhys. Indeed, the numbers of neurons receiving active excitatory AMPA synaptic inputs were about three times higher in the BrainPhys cultures (67% vs 23% of Type 3-5 neurons, **Fig 4i**). Similarly, the numbers of neurons receiving functional inhibitory GABA synaptic inputs were about two times higher in the BrainPhys cultures (42% vs 24%, **Fig 4i**). To further support to these results the proportion of neurons receiving active excitatory synapses in a large unmatched sample of patched neurons was also examined. To clean up the samples only Type 3, 4 and 5 neurons which spent about 1 month in different basal media before being patched in ACSF (n=223 neurons matured in BrainPhys, 69 neurons matured in DMEM) were included. This large sample allowed comparison of each Type with each other and confirmation that maturation of the cells in BrainPhys for ∼4 weeks more than doubles the proportions of neurons with active excitatory synaptic inputs (**Fig 4j**).

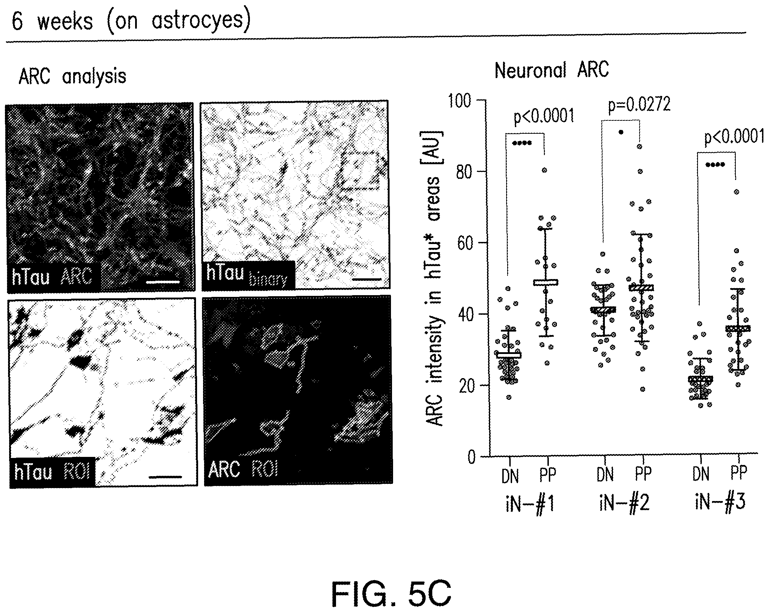

It was then shown that BrainPhys-based medium can also be used with human neurons directly reprogrammed from fibroblasts (hiNs) (**Fig. 5**). In those experiments, the maturation of iNs with BrainPhys based or a mixture (50:50) of DMEM/F12 and Neurobasal with the same supplements (N2, B27, Ascorbic acid, Retinoic acid, Laminin, BDNF, GDNF) was specifically compared. Like others it was found that regardless of the basal medium plating purified iNs neurons on a layer of human or mouse primary astrocytes improved the overall morphological qualities of the cultures. However, it was clear that BrainPhys significantly increased the level of ARC protein expression in the human INs (**Fig 5c****-d**). ARC is an immediate early gene that has been shown to correlate with functional activity of neurons and to be critical for memory formation.

To further characterize the influence of different media on human neuronal cultures, a multi-electrode array (MEA) system comprising 16 electrodes per well of a 48 well plate (Axion) was used. This approach allowed examination of the influence of BrainPhys on neuronal function of the same neurons over several weeks, in comparison to several other conditions used by the research community to culture neurons *in vitro.*

For these experiments pure and thoroughly characterised human iPSC neurons commercially available (iCell neurons from Cellular Dynamics) were used. Since the frozen stock of these neurons are already presumed to be mature neurons, the cells can be thawed directly on the MEA plate and recorded only few days later.

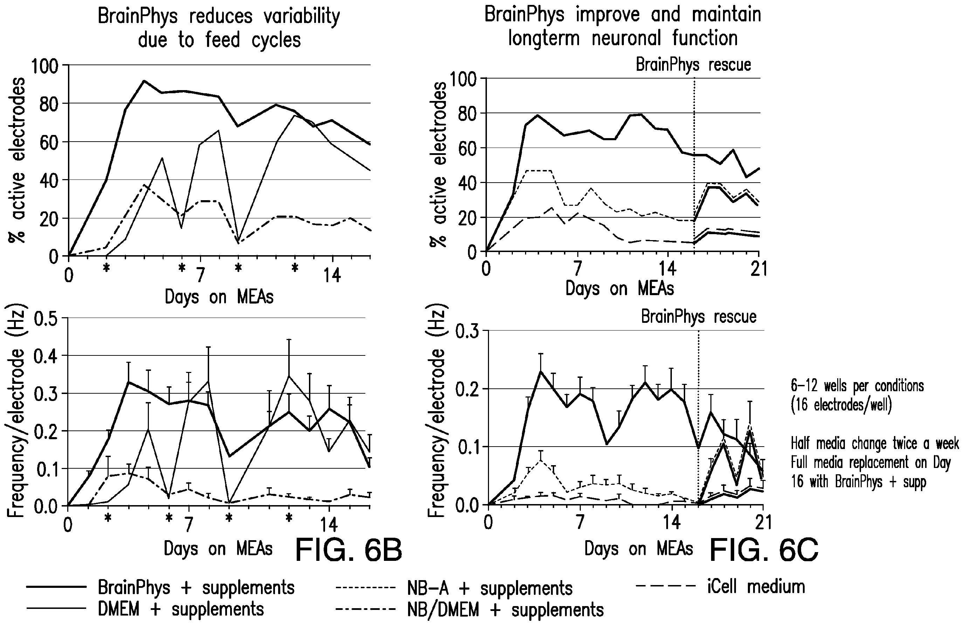

It was first noted that regardless the basal media used (e.g. BrainPhys, NB-A, DMEM/F12, mixture of DMEM/F12 and NB-A) adding serum will dramatically impair the neuronal activity. This could be improved by using serum-free supplement (knockout serum or the supplement cocktails tested with patch-clamping). However, it was noted that when tested with serum-free supplements and Neurobasal-A or a mixture of NB and DMEM/f12 the neuronal activity was relatively low and deteriorated within about a week while the activity in DMEM highly fluctuated in synchrony with the feeding cylces. In contrast, BrainPhys without serum significantly improved the neurophysiological activity of human neurons within a few days, but most importantly, the neuronal function was maintained stable over several weeks. Finally, in most cases the poor neuronal performance in the classic media could be remarkably rescued within a few days when replaced with BrainPhys-based medium (**Fig 6**).

In summary, it has been shown that the newly designed basal medium is closer to conditions of a live brain and therefore allows neurons to be neurophysiologically active *in vitro.* It has ben shown that those changes are not detrimental for neuronal differentiation and survival *in vitro.* To the contrary, it has been found that the neural and synaptic activity seen under the more physiological conditions of BrainPhys can promote the maturation and function of human neurons *in vitro* over time and maintain it.

### Cell density in culture

To assess if BrainPhys might change the overall cell density in culture after several weeks, 36 coverslips containing neurons growing side by side in either medium for on average 35 days (range 21-54 days) were fixed and stained. The same density of DAPI nuclei were found in both groups (DMEM = 1657±239/mm², BrainPhys = 1745±252/mm², Mann Whitney P-value = 0.75). To ask whether BrainPhys might change the proportion of dopaminergic neurons, the number of cells staining positively for tyrosine hydroxylase (TH) on 12 cover slips were quantified, but significant differences were not found (DMEM = 8.7±2.7%, BrainPhys 12±2.5% of TH+/DAPI, *P* = *0.26*).

### BrainPhys is also applicable to rodent neurons

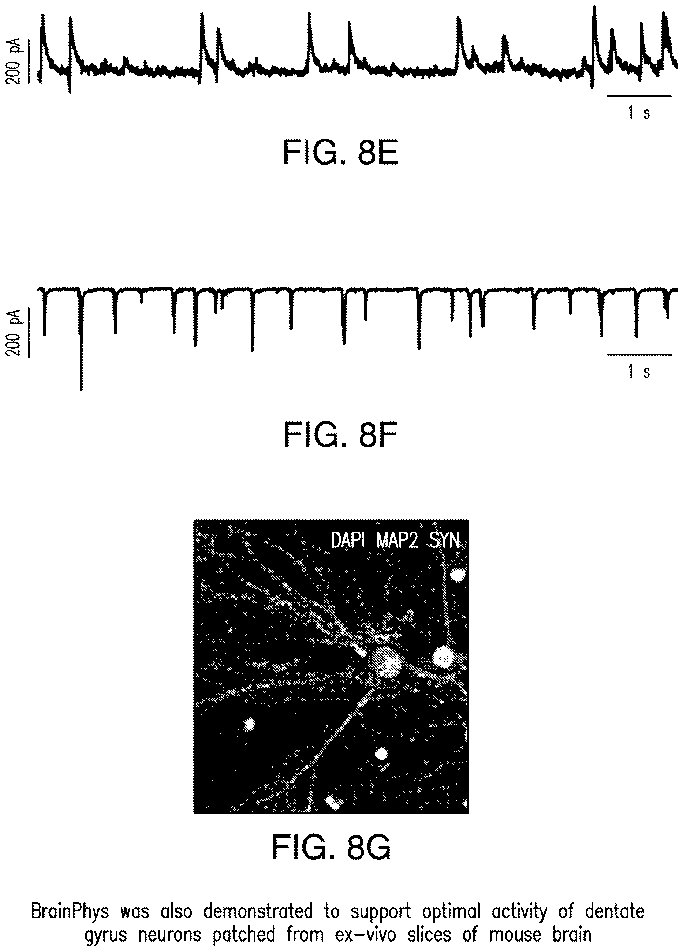

Although BrainPhys was developed to culture human neurons, it was also demonstrated that it could be utilized for electrophysiological recordings in acute mouse brain slices and rat primary neuron cultures. Mouse hippocampal granule cells patched in BrainPhys were fully functional (**Fig. 13**), and their properties recorded in BrainPhys were virtually identical to those in ACSF (n = 4 cells). It was also shown that Rat primary neurons could grow healthfully in BrainPhys (supplemented with sm1) and formed active and functional neural networks within two weeks *in vitro* (**Fig. 8**).

### Discussion

In this Example, basal media were fully tested for their influence on fundamental neuronal functions. It was found that many crucial neurophysiological properties were significantly altered in the widely used DMEM and Neurobasal media (**Table 1**). A new basal medium was therefore designed that was tested on human and rodent neurons. BrainPhys overcame the problems that were identified in DMEM and Neurobasal. With the appropriate supplements, BrainPhys may be used to differentiate NPCs into neurons and provides conditions for mature neurons to actively operate *in vitro.* BrainPhys can also be used acutely with or without supplements to assess electrophysiologically the functional properties of human and rodent neurons in *in vitro* culture or in *ex vivo* brain slices.

### BrainPhys was designed to mimic the human brain physiological environment

Most of the components in BrainPhys are essential for basic cell functioning. BrainPhys is a mixture of 9 inorganic salts, 18 amino acids, 9 vitamins and 5 extra components that include dextrose, sodium pyruvate, Hepes, cholesterol and phenol red (**Table 2**). To allow the neurons to function *in vitro,* as they would in a live brain, the concentrations of many components present in either DMEM or Neurobasal were adapted. For instance, 20 components in DMEM and 39 in Neurobasal were removed, added, or changed by more than 10% in BrainPhys. The concentrations of inorganic salts in BrainPhys resemble the concentrations reported in cerebrospinal fluid. Neurobasal osmolarity is ∼30% lower than neurophysiological levels (∼220 or 250 mOsmol/L for Neurobasal-A). In contrast, the osmolarity of BrainPhys was set to match the osmolarity of physiological cerebrospinal fluid (∼305 mOsmol/L). Several extra components present in DMEM or Neurobasal that were reported as potentially toxic to neurons were reduced or completely removed (e.g, Lipoic acid which may induce oxidative stress, hypoxantine which may reduce Na+K+ atpase and induce oxidative stress, HEPES, riboflavin wich may be phototoxic, L-cysteine hydrochloride which may be neurotoxic and interact with NMDA receptors, folic acid and homocysteine deficiency which may impair DNA repair in neurons and sensitize them to amyloids, Copper which can block extra synaptic GABAa receptors, and ferric sulfate). The concentrations of several amino acids and vitamins were changed to improve neurophysiological functions. The pH of BrainPhys was adjusted to physiological pH (∼7.3-7.5) without perturbing important inorganic salt concentrations. The pH is steadily maintained with pH buffers sensitive to CO₂ levels and low concentrations of Hepes.

In DMEM and Neurobasal, the glucose levels are at least two to five times higher than those in the brain of hyperglycemic patients (Gruetter *et al.*, 1992). To realistically model neurological disorders in a dish, it has been reported to be important to attempt to mimic typical physiological energetic activity in the brain (Zilberter *et al.,* 2010). This is particularly important since several neurological disorders have been related to mitochondria dysfunction (Knott *et al.,* 2008; Cooper *et al.,* 2012; Itoh *et al.,* 2012). In BrainPhys, the energetic components are balanced to provide glycemic levels similar to those reported for the brains of healthy patients. It was also noted that the abnormal levels of ionic currents (Ca2+, Na+, K+, and Cl-) in both DMEM and Neurobasal. Ligand-activated (e.g., AMPA or GABA) or voltage-dependent (e.g., Nav, Kv, Cav) ionotropic channels usually work in tandem with ionic pumps to re-establish resting concentrations of intracellular ions. Ionic pumps use energy (ATP) to work against the osmotic gradients. If a large number of synaptic and extra-synaptic passive ionotropic channels are chronically open, as reported here for DMEM and Neurobasal, the pumps will fight against the gradients in vain, which is likely to waste a considerable amount of cellular energy.

The present example shows that BrainPhys significantly improved the differentiation of cells into functional neurons by combining neurophysiological improvements at various levels, including synaptic function, action potential generation and energetic maintenance.
The pH of BrainPhys was adjusted to physiological pH (∼7.3-7.4) without perturbing important inorganic salt concentrations. The pH is steadily maintained with pH buffers sensitive to ambient CO₂ levels (bicarbonate salts) and low concentrations of Hepes.

Several extra components present in DMEM or Neurobasal that were reported as potentially toxic to neurons were reduced or completely removed (e.g lipoic acid, hypoxantine, HEPES, Roboflavin, L-cysteine hydrochloride, Ferric sulfate, cupric sulfate). The concentrations of several amino acids and vitamins were changed to improve neurophysiological functions.

The present exmaple also shows the abnormal levels of ionic currents (Ca2+, Na+, K+, and Cl-) in both DMEM and Neurobasal. Ligand-activated (e.g., AMPA or GABA) or voltage-dependent (e.g., Nav, Kv, Cav) ionotropic channels that usually work in tandem with ionic pumps to re-establish resting concentrations of intracellular ions. Ionic pumps use energy (ATP) to work against the osmotic gradients. If a large number of synaptic and extra-synaptic passive ionotropic channels are chronically open, as reported here for DMEM and Neurobasal, the pumps will fight against the gradients in vain, which is likely to waste a considerable amount of cellular energy.

Most of the components in BrainPhys are essential for basic cell functioning. BrainPhys is a mixture of 9 inorganic salts, 18 amino acids, 9 vitamins and 5 extra components that include dextrose, sodium pyruvate, cholesterol, Hepes, and phenol red (**Table 2**). To allow the neurons to function *in vitro,* as they would in a live brain, the concentrations of many components present in either DMEM or Neurobasal were adapted. For instance, 20 components in DMEM and 39 in Neurobasal were removed, added, or changed by more than 10% in BrainPhys. It has been shown that BrainPhys significantly improved the differentiation of cells into functional neurons by combining neurophysiological improvements at various levels, including synaptic function, action potential generation and energetic maintenance.

### BrainPhys is adequate for human and rodent neurons

BrainPhys was extensively and successfully tested on different lines of human neurons, including iPSC- and ESC-derived neurons obtained by using different protocols. It was also shown that BrainPhys is adapted for electrophysiological recordings of mouse hippocampal slices and to culture rat primary hippocampal neurons. BrainPhys should also support neurons from a diversity of brain regions and from other species.

### Modelling neurological disorders in a dish: previous work without BrainPhys and future experiments

The present example describes that available basal media, which are used to differentiate human neurons, are very different from conditions in the living brain and, therefore, sub-optimal for the electrical activity of neuronal cultures. One important aspect of optimizing both growth and sustainability of neurons *in vitro,* as with BrainPhys, is that the probability of finding mature and functional neurons is relatively low in current conditions. In addition, most neurological disorders are chronic and progressive and are very closely related to neuronal activity and synaptic function; thus when modelling human neurological and psychiatric disorders *in vitro,* the lack of physiological conditions and activity might introduce artefacts or mask the real mechanisms of the pathologies. Although relevant phenotypes were found between patient and control iPSC-derived neuronal cultures differentiated in current media (Marchetto *et al.,* 2010; Brennand *et al.,* 2011; Nguyen *et al.,* 2011; Seibler *et al.,* 2011; Bellin *et al.,* 2012; Egawa *et al.,* 2012; Israel *et al.,* 2012; Shi *et al.,* 2012), new phenotypes might be revealed from studying neurons in conditions promoting their electrophysiological activity. Neural models closely mimicking the living brain will be more likely to recapitulate the dysfunctions occurring in patients' brains and, in turn, lead to the discovery of more effective treatments against neurological and psychiatric disorders. BrainPhys is advantageous for such use by sustaining physiological neural activity *in vitro.*

### Materials and methods

**Human neurons:** Human dermal fibroblasts were reprogrammed into pluripotent cells with the four Yamanaka factors (Oct3/4, Sox2, Klf4 and cMyc) either in a retroviral vector or a non-integrating Sendai viral vector. Human iPSC and ESC were differentiated into neural progenitors (NPCs) as previously described (Brennand *et al.,* 2011; Boyer *et al.,* 2012). For neural maturation, NPCs were plated on glass coverslips (Fisher Scientific 12-545-80) coated with poly-ornithine (Sigma P3655) and laminin (Invitrogen 23017-015) and cultured in neuronal maturation media (NM see below "Media and extracellular solution") in 24-well plates. Half of the media was gently replaced two to three times a week. The plates were kept in the incubator at 37degC with 5% C02.

**Direct conversion of human dermal fibroblasts into induced neurons (iN):** Primary human dermal fibroblasts HDFs were established from skin biopsies from healthy donors and cultured in DMEM medium containing 15% FBS and 0.1% NEAA (all Gibco). Fibroblast cell line #1 was obtained from ATCC (BJ-CRL-2522™ foreskin fibroblasts); #2 and #3 were obtained from the Coriell Institute (catalog IDs GM22159 and AG08498).

Direct conversion was performed on a Ngn2/Ascl1-based protocol similar to previously described by Ladewig et al. with slight modifications (Ladewig et al. 2012). Coding sequences for human Ascl1 and Ngn2 were linked by a 2A peptide sequence and cloned into pLVX-Tight-Puro construct (Clontech) resulting in the pLVXTP-N2A. Lentiviral particles for pLVX-EtO and pLVXTP-N2A were produced in HEK-293FT cells and enriched by centrifugation. 48h following transduction, transgenic iN-competent fibroblasts were further passaged in the presence of G418 (200 µg/ml Gibco) and puromycin (1 µg/ml; Sigma-Aldrich) in tetracycline-free FBS-containing media.

To generate induced neurons the media was changed to induced neuron conversion (iNC) media based on either DMEM:F12/Neurobasal (DN; 1:1 v/v) or BrainPhys® (BP) for 3 weeks. NC contains the following supplements: N2 supplement and B27 supplement (both 1x; Gibco), doxycycline (2 µg/ml, Sigma-Aldrich), Laminin (1 µg/ml, life technologies), dibutyryl cyclic-AMP (500 µg/ml, Sigma-Aldrich), human recombinant noggin (150 ng/ml; Preprotech), LDN-193189 (5 µM; Cayman Chemical Co) and A83-1 (5 µM; Stemgent), CHIR99021 (3 µM, LC Laboratories), Forskolin (5 µM, LC Laboratories) and SB-431542 (10 µM; Cayman Chemical Co). This results two iNC media DN-iNC and BP-iNC which were changed every 3 days.

To test whether direct iN conversion can be performed in BrainPhys®-based media, iN cultures that converted for 3 weeks in either DN-iNC or BP-iNC were fixed with 4% PFA and stained for the neuronal markers beta-III-tubulin (rabbit, 1:3000, Millipore), hTau (PHF1, mouse, 1:500, kind gift from Peter Davies), NeuN (mouse, 1:100, Millipore) and Map2ab (chicken, 1:300, Abcam).

For full functional maturation, iN cells were gently dislodged from their conversion plate using TrypLE and relocated on top of a monolayer culture of mouse astrocytes (see Mertens et al. AJP 2013) and further cultured in induced neuron maturation (iNM) media containing the supplements GDNF, BDNF (both 20 ng/ml, R&D), dibutyryl cyclic-AMP (500 µg/ml, Sigma-Aldrich), doxycycline (2 µg/ml, Sigma-Aldrich) and laminin (1 µg/ml, life technologies). NM-containing media were again either based on DMEM:F12/Neurobasal (DN; 1:1 v/v) or BrainPhys® (BP), resulting in DN-iNM or BP-iNM which were changed every 3 days. Following 3 weeks of maturation (6 weeks of conversion in total), cultures were fixed with 4% PFA and stained for hTau and ARC (rabbit, 1:200, xyz).

**Rodent neurons:** Rat hippocampal dissociated cultures were prepared at embryonic day 18. Hippocampal dissection were briefly treated with Papain, then gently dissociated and plated in Neurobasal + Neurocult sml neuronal supplement (Cat# 07511, Stem Cell Technologies, USA) on glass coverslips coated with Poly-L-Lysine. To test BrainPhys directly on the brain *ex vivo* rather than in artificial cultures, we prepared 300-µm thick slices of the mouse hippocampus (C57B16).

**Media and extracellular solutions:** Neural Maturation media (NM) consisted of basal media and serum-free supplements. We used the following basal media: BrainPhys (BP, custom-made), DMEM/F12 (DMEM, Gibco, Cat No. 10565-018), Neurobasal-A (NB, Gibco, Cat# 10888-022) or a mixture of DMEM/F12 and Neurobasal-A (DM, 50:50). Most of the DMEM used in these experiments was obtained from Life Technologies; a few batches were custom-made in house. Neurobasal was always obtained from Life Technologies. ACSF and BrainPhys were custom-made in house.

Maturation of neurons was performed in the basal media with the following supplements were added: 1x N2 (Gibco, Cat No. 17502-048), 1x B27 (Gibco, Cat No. 17504-044), Brain-derived Neurotrophic Factor (BDNF, 20 ng/ml; Peprotech, Cat No. 450-02), Glia-derived Neurotrophic Factors (GDNF, 20 ng/ml; Peprotech, Cat No. 450-10), ascorbic acid (AA, 200 nM; Sigma, Cat No. A0278), dibutyryl cyclic AMP (cAMP, ImM Sigma, Cat No. D0627) and laminin (1 µg/ml; Invitrogen, Cat No 23017-015). Acute experiments to measure functional activity were performed in ACSF or in basal media with or without supplements.

**Patch-clamping:** For whole-cell patch-clamp recordings, individual coverslips were transferred into a heated recording chamber and continuously perfused (1 ml min⁻¹) with either basal media or artificial cerebrospinal fluid (ACSF) bubbled with a mixture of CO₂ (5%) and O₂ (95%) and maintained at 25 °C. The composition of ACSF was adjusted to match the inorganic salt concentration and osmolarity of the DMEM and BrainPhys when acutely compared. ACSF contained (in mM) 121 NaCl, 4.2 KCl, 1.1 CaCl2, 1 MgSO4 (or 0.4 MgSO4 and 0.3 MgCl), 29 NaHCO3, 0.45 NaH2PO4-H2O, 0.5 Na2HPO4 and 20 glucose (all chemicals from Sigma).

For targeted whole-cell recordings, we used a 40X water-immersion objective, differential interference contrast filters (all Olympus), an infrared digital camera (Rolera XR -Qimaging), a digidata 1440A/ Multiclamp 700B and Clampex 10.3 (Molecular devices). The resistance of the patch electrodes was between 3 and 5 MOhm. Patch electrodes were filled with internal solutions containing 130 mM K-gluconate, 6 mM KCl, 4 mM NaCl, 10mM Na-HEPES, 0.2 mM K-EGTA; 0.3mM GTP, 2mM Mg-ATP, 0.2 mM cAMP, 10mM D-glucose, 0.15% biocytin and 0.06% rhodamine. The pH and osmolarity of the internal solution were close to physiological conditions (pH 7.3, 290-300 mOsmol/L). Data were all corrected for liquid junction potentials (10 mV). Electrode capacitances were compensated on-line in cell-attached mode (∼7 pF). Recordings were low-pass filtered at 2 kHz, digitized, and sampled at intervals of 50 ms (20 kHz). To control the quality and the stability of the recordings throughout the experiments, access resistance, capacitance and membrane resistance were continuously monitored on-line and recorded. The resistance of the glass pipettes we used was ∼5 MOhm. The access resistance of the cells in our sample was ∼40 MOhm. Patch-clamping results of every tested solution were confirmed by recovery to level comparable to the control. Spontaneous synaptic AMPA events were recorded at the reversal potential of Cl- and could be reversibly blocked by AMPA receptor antagonist (10 uM NBQX, Sigma Ref#N183). Spontaneous synaptic GABA events were recorded at the reversal potential of Na+ and could be reversibly blocked with GABAa receptor antagonist (10 uM SR95531, Sigma Ref#S106). Across all experiments, 292 neurons were patched and analyzed.

**Multi-well MEA plates:** Microelectrode array (MEA) plates were composed of 48 wells with each well containing an array of 16 individual embedded nano-textured gold microelectrodes (∼40-50 µm diameter; 350 µm center-to-center spacing) with 4 integrated ground electrodes, for a total of 768 channels (Axion Biosystems). Prior to culture, each well was coated with 200 µl of a filter-sterilized 0.1% solution of polyethylenimine (Sigma Aldrich) in borate buffer (3.10 g of boric acid (Fisher Scientific) and 4.75 g of sodium tetraborate (Sigma Aldrich) in 11 of distilled water) at room temperature. The solution was removed after 1 h, and the wells were washed 4 times with sterile water and air-dried overnight in a sterile biological safety cabinet.

**Cell culture on MEAs:** One vial of cryopreserved human iPSC-derived iCell neurons (Cellular Dynamics International) was thawed and pelleted by centrifugation according to the manufacturer's protocol, and resuspended at a concentration of 28,000 viable neurons/µl in iCell Neuron Maintenance Medium with 10 µg/ml laminin (Sigma Aldrich). A 5 µl droplet of the cell suspension (140,000 neurons) was added to the center of each well in the MEA, directly over the electrode area. Sterile water was added to the area around the wells to reduce evaporation, and the MEA was incubated with the seeded neurons in a cell culture incubator at 37°C and 5% CO₂ for 1 hour. 300 µl of iCell Neuron Maintenance Medium was then gently added to each well, and the water surrounding the wells was removed. 2 days after the cell plating (day in vitro (DIV 2)) the medium was replaced with one of 8 different media: Neurobasal-A (Life Technologies) with 10% defined fetal bovine serum (FBS; HyClone); BrainPhys with 10% FBS; DMEM/F12 (Life Technologies) with 10% FBS; Neurobasal-A supplemented with N2 (Gibco), B27 (Gibco), BDNF (20 ng/ml; Peprotech), GDNF (20 ng/ml; Peprotech), ascorbic acid (200 nM; Sigma), cAMP (1mM; Sigma), and laminin (1 ug/ml; Life Technologies); BrainPhys with the aforementioned supplements; DMEM/F12 (Life Technologies) with the aforementioned supplements; a 1:1 mixture of Neurobasal-A/DMEM with the aforementioned supplements; or fresh iCell Neuron Maintenance Medium (6 wells each medium). At DIV 6, 9, and 13, 50% of the medium in each well was exchanged with fresh medium. On DIV 16, all medium was replaced with supplemented BrainPhys.

**MEA recording and data analysis:** Between DIV 2 and 21, spontaneous activity was recorded for 10 min each day at 37 °C using the Maestro MEA system (Axion Biosystems) and the associated Axion Integrated Studio (AxIS 1.8.1.5). The 768 channels of the MEA were sampled simultaneously with a gain of 1200X and a sampling rate of 12.5 kHz/channel. For all recordings, a Butterworth band-pass filter (200 Hz - 3 kHz) was applied along with an adaptive threshold spike detector set at 6× standard deviation. Data from the recordings were saved to 3 different file types simultaneously; a raw data file (*.raw file) that included all data, a spike counts file (*.csv file) that included the spikes per electrode with a 1 s bin time, and an alpha map (*.map file) that included spike timing and profile information. The frequency reported in the analysis represent the average frequency of 96 to 192 electrodes (6 to 12 wells) for each tested condition. The percentage of active electrode represent the number of electrode with a spike frequency >0.005 Hz.

**Analysis and statistics:** Statistical analysis of electrophysiology data and calcium imaging was assisted with Clampfit 10.3, Matlab 2011b, Igor Pro 6, Prism 5, MiniAnalyis and Microsoft Excel. Standard errors of the mean were reported. Statistical significance was assessed with two-tailed non-parametric paired (Wilcoxon) or unpaired (Mann Whitney) tests.

**Functional types classification:** "Type 0 cells" did not express voltage-dependent sodium currents and were excluded from analysis. "Type 1 neurons" expressed small Nav currents but were not able to fire action potentials above -10 mV. The arbitrary limit of -10 mV was chosen as it is close to the reversal potential of Na+ (0 mV). Healthy action potentials usually reach or overshoot the reversal potential of Na+. "Type 2 neurons" fired one action potential above -10 mV, which was followed by a plateau. "Type 3 neurons" also fired one action potential above -10 mV and one or few aborted spikes below -10 mV. "Type 4 neurons" fired more than one action potential above -10 mV but at a frequency below 10 Hz. "Type 5 neurons" fired action potentials above -10 mV at 10 Hz or more. Our categorization of functional types of neurons followed a continuum that might relate to the stage of maturity of the neurons. While Type 1 neurons would be considered immature, Type 5 neurons might be considered more mature and functional. Interestingly, we found every functional type of cell at most differentiation time points we looked at (range of two to six weeks old). This finding suggests a high degree of variability in the functional maturity of neurons even within cultures of the same age. Remarkably, in our samples, the large majority of cells receiving active excitatory synapses were Type 5 neurons and we found no spontaneously active AMPA and GABA synaptic inputs in Type 1-2 neurons.

**Calcium imaging:** Neurons attached on glass coverslips were incubated for ∼20 min (37 °C, 5% CO₂, and 95% humidity) with 4 uM calcium-sensitive dye Fluo-4 AM (Invitrogen, Cat No. F14201) in neural differentiating media. Fluo-4 was washed and coverslips were transferred into a heated recording chamber and continuously perfused (1 ml/min) with either basal media or ACSF bubbled with a mixture of CO₂ (5%) and O₂ (95%) and maintained in the chamber at 35 °C. We waited at least 15 min after the coverslip transfer before starting any recording. Calcium imaging movies were acquired with a laser-scanning microscope at 471 nm (Olympus, Fluoview FV1000MPE) and a 25X objective (Olympus, XLPLN NA 1.05). At least two consecutive movies of 5 min were recorded in each condition (control, tested medium, recovery). In many cases the extracellular medium defined as control and recovery medium was ACSF but similar results were obtained when the order of media perfusion was changed (e.g, control=BrainPhys, tested=ACSF, Recovery=BrainPhys). When necessary, gain sensitivity and focus were adjusted between movies. Laser power was not changed (∼2%). After changing each perfusate, we also waited at least 5 mins to make sure the entire bath solution was replaced before recordings. We analyzed no more than 3 fields of view per coverslip.

Regions of interest (ROIs) were categorized as spontaneously active when at least one clear neuronal calcium event was detected on a soma. Neuronal calcium events were defined as a sharp transient increase in fluorescence intensity (Fluo-4 AM, dF/F >5%, fast rise, slower decay). The time series for each ROI were calculated with the Olympus Fluoview FV1000 software.

**Immunohistochemistry:** Immunohistochemistry experiments were performed on neurons plated on glass coverslips in 24-well plates. Comparison between media conditions were made on the same cell line, growing side by side in the same plate with the same original cell density. Standard immunohistochemistry protocols were used. Coverslips were stained with DAPI and a combination of the following antibodies: mouse-Map2(2a+2b) (1:500, Sigma), mouse-TUJ1 (1:1000, Covance), Rabbit-Synapsinl (1:500, Calbiochem), Rabbit-TH (1:500, Pel-Freez), Rabbit-GFAP (1:200, Dako), rabbit-GABA (1:500, Sigma), Goat-DCX (1:500, Santa Cruz).

### References

Bellin M, Marchetto MC, Gage FH, Mummery CL (2012) Induced pluripotent stem cells: the new patient? Nature Reviews Molecular Cell Biology 13:713-726.
Boulting GL, Kiskinis E, Croft GF, Amoroso MW, Oakley DH, Wainger BJ, Williams DJ, Kahler DJ, Yamaki M, Davidow L, Rodolfa CT, Dimos JT, Mikkilineni S, MacDermott AB, Woolf CJ, Henderson CE, Wichterle H, Eggan K (2011) A functionally characterized test set of human induced pluripotent stem cells. Nat Biotechnol 29:279-286.
Boyer LF, Campbell B, Larkin S, Mu Y, Gage FH (2012) Dopaminergic differentiation of human pluripotent cells. Curr Protoc Stem Cell Biol Chapter 1:Unit1H.6.
Bráz JM, Sharif-Naeini R, Vogt D, Kriegstein A, Alvarez-Buylla A, Rubenstein JL, Basbaum AI (2012) Forebrain GABAergic neuron precursors integrate into adult spinal cord and reduce injury-induced neuropathic pain. Neuron 74:663-675.
Brennand KJ, Simone A, Jou J, Gelboin-Burkhart C, Tran N, Sangar S, Li Y, Mu Y, Chen G, Yu D, McCarthy S, Sebat J, Gage FH (2011) Modelling schizophrenia using human induced pluripotent stem cells. Nature.
Brewer GJ, Torricelli JR, Evege EK, Price PJ (1993) Optimized survival of hippocampal neurons in B27-supplemented neurobasal?, a new serum-free medium combination. J Neurosci Res 35:567-576.
Caiazzo M, Dell'Anno MT, Dvoretskova E, Lazarevic D, Taverna S, Leo D, Sotnikova TD, Menegon A, Roncaglia P, Colciago G, Russo G, Carninci P, Pezzoli G, Gainetdinov RR, Gustincich S, Dityatev A, Broccoli V (2011) Direct generation of functional dopaminergic neurons from mouse and human fibroblasts. Nature 476:224-227.
Cattaneo E, McKay R (1990) Proliferation and differentiation of neuronal stem cells regulated by nerve growth factor. Nature 347:762-765.
Cooper O et al. (2012) Pharmacological Rescue of Mitochondrial Deficits in iPSC-Derived Neural Cells from Patients with Familial Parkinson's Disease. Sci Transl Med 4:141ra90-141ra90.
Davson H, Segal MB (1996) Physiology of the CSF and blood-brain barriers.
Egawa N et al. (2012) Drug screening for ALS using patient-specific induced pluripotent stem cells. Sci Transl Med 4:145ra104.
Eiraku M, Takata N, Ishibashi H, Kawada M, Sakakura E, Okuda S, Sekiguchi K, Adachi T, Sasai Y (2011) Self-organizing optic-cup morphogenesis in three-dimensional culture. Nature 472:51-56.
Giorgetti A, Marchetto MCN, Li M, Yu D, Fazzina R, Mu Y, Adamo A, Paramonov I, Cardoso JC, Monasterio MB, Bardy C, Cassiani-Ingoni R, Liu G-H, Gage FH, Izpisua Belmonte JC (2012) Cord blood-derived neuronal cells by ectopic expression of Sox2 and c-Myc. Proc Natl Acad Sci USA 109:12556-12561.
Gruetter R, Novotny EJ, Boulware SD, Rothman DL, Mason GF, Shulman GI, Shulman RG, Tamborlane WV (1992) Direct measurement of brain glucose concentrations in humans by 13C NMR spectroscopy. Proc Natl Acad Sci USA 89:1109-1112.
Hermann A, Gastl R, Liebau S, Popa MO, Fiedler J, Boehm BO, Maisel M, Lerche H, Schwarz J, Brenner R, Storch A (2004) Efficient generation of neural stem cell-like cells from adult human bone marrow stromal cells. J Cell Sci 117:4411-4422.
Israel MA, Yuan SH, Bardy C, Reyna SM, Mu Y, Herrera C, Hefferan MP, Van Gorp S, Nazor KL, Boscolo FS, Carson CT, Laurent LC, Marsala M, Gage FH, Remes AM, Koo EH, Goldstein LSB (2012) Probing sporadic and familial Alzheimer's disease using induced pluripotent stem cells. Nature.
Itoh K, Nakamura K, Iijima M, Sesaki H (2012) Mitochondrial dynamics in neurodegeneration. Trends Cell Biol.
Johnson M, Weick J, Pearce R (2007) Functional Neural Development from Human Embryonic Stem Cells: Accelerated Synaptic Activity via Astrocyte Coculture. The Journal of Neuroscience.
Knott AB, Perkins G, Schwarzenbacher R, Bossy-Wetzel E (2008) Mitochondrial fragmentation in neurodegeneration. Nat Rev Neurosci 9:505-518.
Koch P, Opitz T, Steinbeck JA, Ladewig J, Brüstle O (2009) A rosette-type, self-renewing human ES cell-derived neural stem cell with potential for in vitro instruction and synaptic integration. Proc Natl Acad Sci USA 106:3225-3230.
Kriks S, Shim J-W, Piao J, Ganat YM, Wakeman DR, Xie Z, Carrillo-Reid L, Auyeung G, Antonacci C, Buch A, Yang L, Beal MF, Surmeier DJ, Kordower JH, Tabar V, Studer L (2011) Dopamine neurons derived from human ES cells efficiently engraft in animal models of Parkinson's disease. Nature.
Li X-J, Du Z-W, Zarnowska ED, Pankratz M, Hansen LO, Pearce RA, Zhang S-C (2005) Specification of motoneurons from human embryonic stem cells. Nat Biotechnol 23:215-221.
Marchetto MC, Brennand KJ, Boyer LF, Gage FH (2011) Induced pluripotent stem cells (iPSCs) and neurological disease modeling: progress and promises. Hum Mol Genet 20:R109-R115.
Marchetto MC, Carromeu C, Acab A, Yu D, Yeo GW, Mu Y, Chen G, Gage FH, Muotri AR (2010) A model for neural development and treatment of Rett syndrome using human induced pluripotent stem cells. Cell 143:527-539.
Nguyen HN, Byers B, Cord B, Shcheglovitov A, Byrne J, Gujar P, Kee K, Schüle B, Dolmetsch RE, Langston W, Palmer TD, Pera RR (2011) LRRK2 mutant iPSC-derived DA neurons demonstrate increased susceptibility to oxidative stress. Cell Stem Cell 8:267-280.
Okabe S, Forsberg-Nilsson K, Spiro AC, Segal M, McKay RD (1996) Development of neuronal precursor cells and functional postmitotic neurons from embryonic stem cells in vitro. Mech Dev 59:89-102.
Pang ZP, Yang N, Vierbuchen T, Ostermeier A, Fuentes DR, Yang TQ, Citri A, Sebastiano V, Marro S, Südhof TC, Wemig M (2011) Induction of human neuronal cells by defined transcription factors. Nature 476:220-223.
Pfisterer U, Kirkeby A, Torper O, Wood J, Nelander J, Dufour A, Bjorklund A, Lindvall O, Jakobsson J, Parmar M (2011) Direct conversion of human fibroblasts to dopaminergic neurons. Proc Natl Acad Sci USA 108:10343-10348.
Qiang L, Fujita R, Yamashita T, Angulo S, Rhinn H, Rhee D, Doege C, Chau L, Aubry L, Vanti WB, Moreno H, Abeliovich A (2011) Directed conversion of Alzheimer's disease patient skin fibroblasts into functional neurons. Cell 146:359-371.
Seibler P, Graziotto J, Jeong H, Simunovic F, Klein C, Krainc D (2011) Mitochondrial Parkin Recruitment Is Impaired in Neurons Derived from Mutant PINK1 Induced Pluripotent Stem Cells. J Neurosci 31:5970-5976.
Shao W, Zhang S-Z, Tang M, Zhang X-H, Zhou Z, Yin Y-Q, Zhou Q-B, Huang Y-Y, Liu Y-J, Wawrousek E, Chen T, Li S-B, Xu M, Zhou J-N, Hu G, Zhou J-W (2012) Suppression of neuroinflammation by astrocytic dopamine D2 receptors via αB-crystallin. Nature.
Shi Y, Kirwan P, Smith J, MacLean G, Orkin SH, Livesey FJ (2012) A human stem cell model of early Alzheimer's disease pathology in Down syndrome. Sci Trans1 Med 4:124ra29.
Soldner F et al. (2011) Generation of isogenic pluripotent stem cells differing exclusively at two early onset Parkinson point mutations. Cell 146:318-331.
Soldner F, Hockemeyer D, Beard C, Gao Q, Bell GW, Cook EG, Hargus G, Blak A, Cooper O, Mitalipova M, Isacson O, Jaenisch R (2009) Parkinson's Disease Patient-Derived Induced Pluripotent Stem Cells Free of Viral Reprogramming Factors. Cell 136:964-977.
Son EY, Ichida JK, Wainger BJ, Toma JS, Rafuse VF, Woolf CJ, Eggan K (2011) Conversion of mouse and human fibroblasts into functional spinal motor neurons. Cell Stem Cell 9:205-218.
Takahashi K, Tanabe K, Ohnuki M, Narita M, Ichisaka T, Tomoda K, Yamanaka S (2007) Induction of Pluripotent Stem Cells from Adult Human Fibroblasts by Defined Factors. Cell 131:861-872.
Vierbuchen T, Ostermeier A, Pang ZP, Kokubu Y, Südhof TC, Wernig M (2010) Direct conversion of fibroblasts to functional neurons by defined factors. Nature 463:1035-1041.
Vilchez D, Boyer L, Morantte I, Lutz M, Merkwirth C, Joyce D, Spencer B, Page L, Masliah E, Berggren WT, Gage FH, Dillin A (2012) Increased proteasome activity in human embryonic stem cells is regulated by PSMD11. Nature 489:304-308.
Walz W, Boulton A, Baker GB (2002) Patch-clamp Analysis: Advanced Techniques.
Zilberter Y, Zilberter T, Bregestovski P (2010) Neuronal activity in vitro and the in vivo reality: the role of energy homeostasis. Trends Pharmacol Sci 31:394-401.

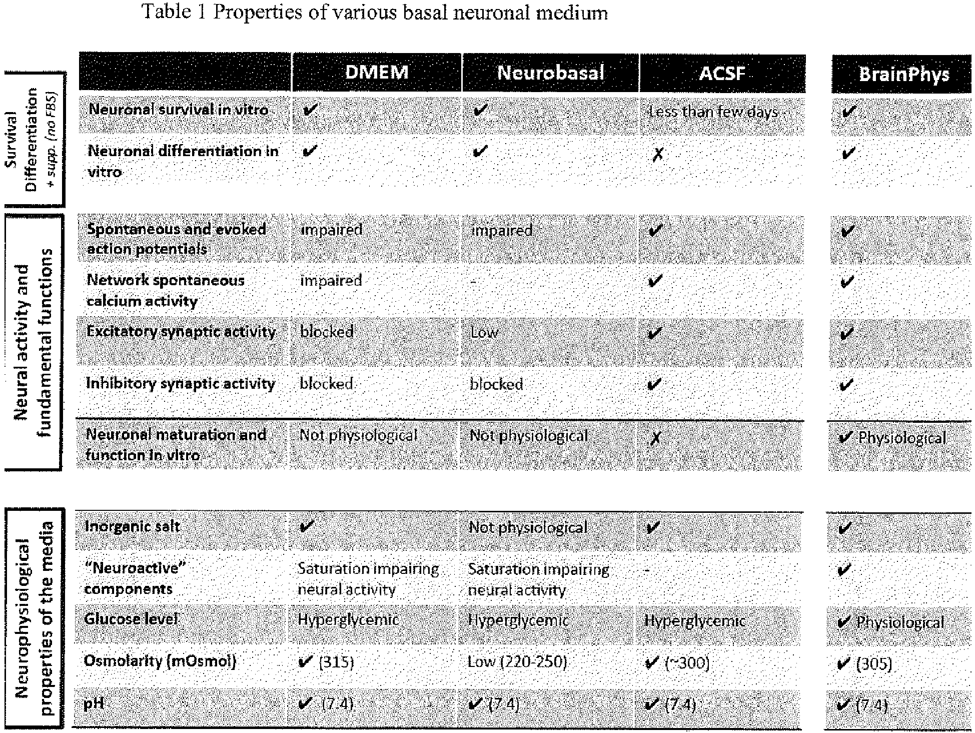

### Table 2. Components in BrainPhys (CB2) basal medium

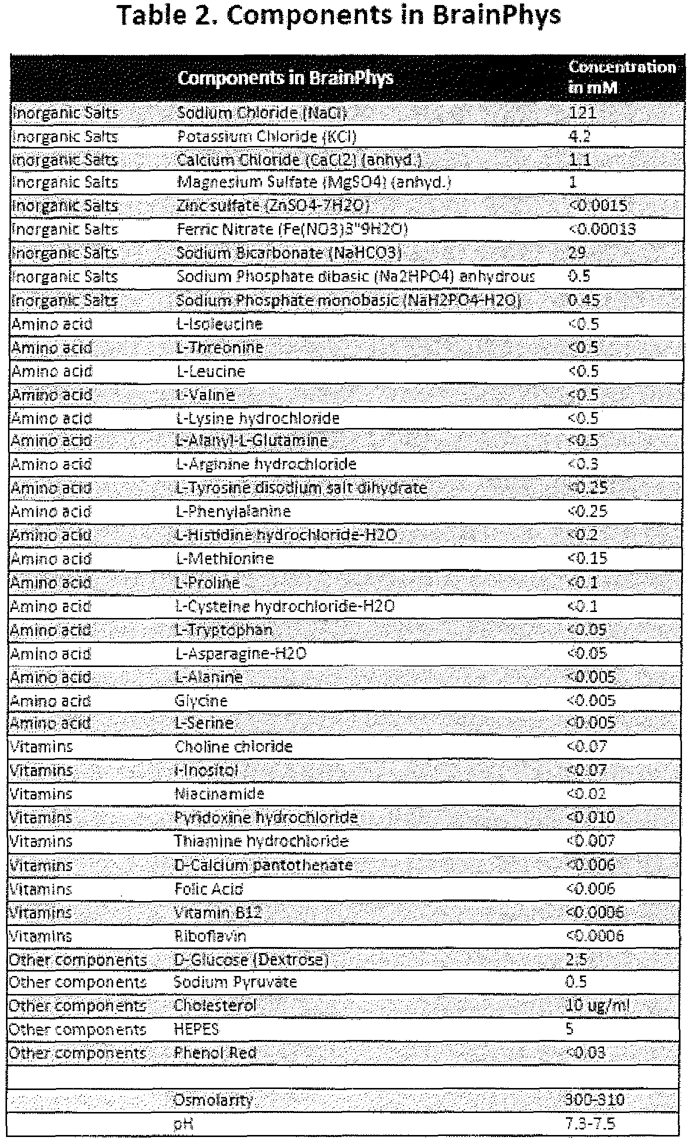

**Table 3. Comparison of BrainPhys with other media**

| | **BrainPhys medium** | **Ingredient function** | **Ingredient class** | **Imgredient** | **BrainPhys(CB2) mM** | ***Ranges of BrainPhys (CB2) mM*** | **Neurobasal-A (mM)** | **Neurobasal (mM)** | **DMEM/F12 Glutamax #10565 (mM)** |
|---|---|---|---|---|---|---|---|---|---|
| **0** | BrainPhys Basal | neuroactive | Inorganic Salt | Sodium Chloride (NaCl) | 121.00000000 | >*70 and <150* | 68.97000000 | 51.72000000 | 120.68000000 |
| **0.1** | BrainPhys Basal | neuroactive | Inorganic Salt | Potassium Chloride (KCl) | 4.20000000 | <5 | 5.33000000 | 5.33000000 | 4.16000000 |
| **0.2** | BrainPhys Basal | neuroactive | Inorganic Salt | Calcium Chloride (CaCl2) (anhyd.) | 1.10000000 | *>0.8 and <2* | 1.80000000 | 1.80000000 | 1.05000000 |
| **0.3** | BrainPhys Basal | neuroactive | Inorganic Salt | Magnesium Sulfate (MgSO4) (anhyd.) | 1.00000000 | <*2* | 0.00000000 | 0.00000000 | 0.40700000 |
| 0.4 | BrainPhys Basal | neuroactive | Inorganic Salt | Magnesium Chloride (anhydrous) | 0.00000000 | <*2* | 0.81400000 | 0.81400000 | 0.30100000 |
| 0.5 | BrainPhys Basal | neuroactive | Inorganic Salt | Ferric Nitrate (Fe(NO3)3"9H2O) | 0.00012400 | *<0.0004* | 0.00024800 | 0.00024800 | 0.00012400 |
| 0.6 | BrainPhys Basal | neuroactive | Inorganic Salt | Zinc sulfate (ZnSO₄-7H₂O) | 0.00150000 | *<0.002* | 0.00067400 | 0.00067400 | 0.00150000 |
| 1 | BrainPhys Basal | neuroactive | Inorganic Salt | Cupric sulfate (CuSO4-5H2O) | 0.00000000 | *<0.00001* | 0.00000000 | 0.00000000 | 0.00000520 |
| 1 | BrainPhys Basal | neuroactive | Inorganic Salt | Ferric sulfate (FeSO4-7H2O) | 0.00000000 | *<0.0015* | 0.00000000 | 0.00000000 | 0.00150000 |
| 1 | BrainPhys Basal | pH | Inorganic Salt | Sodium Bicarbonate (NaHCO3) | 29.00000000 | <*35* | 26.19000000 | 26.19000000 | 29.02000000 |
| 1 | BrainPhys Basal | pH | Inorganic Salt | Sodium Phosphate dibasic (Na2HPO4) anhydrous | 0.50000000 | <*1* | 0.00000000 | 0.00000000 | 0.50000000 |

| | **BrainPhys medium** | **Ingredient function** | **Ingredient class** | **Imgredient** | **BrainPhys(CB2) mM** | ***Ranges of BrainPhys (CB2) mM*** | **Neurobasal-A (mM)** | **Neurobasal (mM)** | **DMEM/F12, Glutamax #10565 (mM)** |
|---|---|---|---|---|---|---|---|---|---|
| 1 | BrainPhys Basal | pH | Inorganic Salt | Sodium Phosphate monobasic (NaH2PO4-H2O) | 0.45000000 | *<1* | 0.90600000 | 0.90600000 | 0.45300000 |
| 2 | BrainPhys Basal | neuroactive | Amino acid | Glycine | 0.00200000 | *<0.05* | 0.40000000 | 0.40000000 | 0.25000000 |
| 2 | BrainPhys Basal | neuroactive | Amino acid | L-Alanine | 0.00200000 | *<0.05* | 0.02250000 | 0.02250000 | 0.05000000 |
| 2 | BrainPhys Basal | neuroactive | Amino acid | L-Aspartic acid | 0.00000000 | *<0*.*003* | 0.00000000 | 0.00000000 | 0.05000000 |
| 2 | BrainPhys Basal | neuroactive | Amino acid | L-Glutamic Acid | 0.00000000 | *<0.02* | 0.00000000 | 0.00000000 | 0.05000000 |
| 2 | BrainPhys Basal | neuroactive | Amino acid | L-Serine | 0.00200000 | *<0.03* | 0.40000000 | 0.40000000 | 0.25000000 |
| 3 | BrainPhys Basal | | Amino acid | L-Alanyl-L-Glutamine | 0.50000000 | <2.5 | 0.00000000 | 0.00000000 | 2.50000000 |
| 3 | BrainPhys Basal | | Amino acid | L-Arginine hydrochloride | 0.30000000 | *<0.5* | 0.39800000 | 0.39800000 | 0.69900000 |
| 3 | BrainPhys Basal | | Amino acid | L-Asparagine-H2O | 0.05000000 | *<0.05* | 0.00553000 | 0.00553000 | 0.05000000 |
| 3 | BrainPhys Basal | | Amino acid | L-Cysteine hydrochloride-H2O | 0.10000000 | *<0.15* | 0.26000000 | 0.26000000 | 0.09980000 |
| 3 | BrainPhys Basal | | Amino acid | L-Cystine 2HCl | 0.00000000 | 0.00000000 | 0.00000000 | 0.00000000 | 0.10000000 |
| 3 | BrainPhys Basal | | Amino acid | L-Histidine hydrochloride-H2O | 0.15000000 | *<0.2* | 0.20000000 | 0.20000000 | 0.15000000 |
| 3 | BrainPhys Basal | | Amino acid | L-Isoleucine | 0.41600000 | *<0.8* | 0.80200000 | 0.80200000 | 0.41600000 |

| | **BrainPhys medium** | **Ingredient function** | **Ingredient class** | **Imgredient** | **BrainPhys (CB2) mM** | ***Ranges of BrainPhys (CB2) mM*** | **Neurobasal-A (mM)** | **Neurobasal (mM)** | **DMEM/F12, Glutamax #10565 (mM)** |
|---|---|---|---|---|---|---|---|---|---|
| 3 | BrainPhys Basal | | Amino acid | L-Leucine | 0.45100000 | *<0.8* | 0.80200000 | 0.80200000 | 0.45100000 |
| 3 | BrainPhys Basal | | Amino acid | L-Lysine hydrochloride | 0.49900000 | *<0.8* | 0.79800000 | 0.79800000 | 0.49900000 |
| 3 | BrainPhys Basal | | Amino acid | L-Methionine | 0.11600000 | *<0.2* | 0.20100000 | 0.20100000 | 0.11600000 |
| 3 | BrainPhys Basal | | Amino acid | L-Phenylalanine | 0.21500000 | <*0.4* | 0.40000000 | 0.40000000 | 0.21500000 |
| 3 | BrainPhys Basal | | Amino acid | L-Proline | 0.06000000 | <*0.1* | 0.06750000 | 0.06750000 | 0.15000000 |
| 3 | BrainPhys Basal | | Amino acid | L-Threonine | 0.44900000 | <*0.7* | 0.79800000 | 0.79800000 | 0.44900000 |
| 3 | BrainPhys Basal | | Amino acid | L-Tryptophan | 0.04410000 | <*0.07* | 0.07840000 | 0.07840000 | 0.04420000 |
| 3 | BrainPhys Basal | | Amino acid | L-Tyrosine disodium salt dihydrate | 0.21400000 | <*0.4* | 0.39800000 | 0.39800000 | 0.21400000 |
| 3 | BrainPhys Basal | | Amino acid | L-Valine | 0.45200000 | *<0.7* | 0.80300000 | 0.80300000 | 0.45200000 |
| 4 | BrainPhys Basal | Energy | Energetic substrate | D-Glucose (Dextrose) | 2.50000000 | *<5* | 25.00000000 | 25.00000000 | 17.51000000 |
| 4 | BrainPhys Basal # | Energy | Energetic substrate | Sodium Pyruvate | 0.50000000 | <*1* | 0.22700000 | 0.22700000 | 0.50000000 |
| 5 | BrainPhys Basal | | Vitamin | Choline chloride | 0.06410000 | <*0.07* | 0.02860000 | 0.02860000 | 0.06410000 |
| 5 | BrainPhys Basal | | Vitamin | D-Calcium pantothenate (B5) | 0.00470000 | *<0.006* | 0.00839000 | 0.00839000 | 0.00470000 |
| 5 | BrainPhys Basal | | Vitamin | Folic Acid (B9) | 0.00601000 | *<0.006* | 0.00907000 | 0.00907000 | 0.00601000 |
| 5 | BrainPhys Basal | | Vitamin | i-Inositol | 0.07000000 | *<0.07* | 0.04000000 | 0.04000000 | 0.07000000 |
| 5 | BrainPhys Basal | | Vitamin | Niacinamide (B3) | 0.01660000 | *<0.02* | 0.03280000 | 0.03280000 | 0.01660000 |
| 5 | BrainPhys Basal | | Vitamin | Pyridoxine hydrochloride | 0.00986000 | <*0.010* | 0.01960000 | 0.01960000 | 0.00986000 |
| 5 | BrainPhys Basal | | Vitamin | Thiamine hydrochloride | 0.00644000 | *<0.007* | 0.01190000 | 0.01190000 | 0.00644000 |
| 5 | BrainPhys Basal | | Vitamin | Vitamin B12 (cyanocobalamin) | 0.00050200 | <*0.0006* | 0.00000500 | 0.00000500 | 0.00050200 |
| 5 | BrainPhys Basal ^{o} | | Vitamin | Riboflavin (B2) | 0.00058200 | <*0.0006* | 0.00106000 | 0.00106000 | 0.00058200 |
| 6 | | | | | | | | | |
| 6.1 | BrainPhys Basal ^{o} | pH | pH buffer | HEPES | 5.00000000 | <*10* | 10.92000000 | 10.92000000 | 0.00000000 |
| 6.2 | BrainPhys Basal ^{a} | pH | pH Indicator | Phenol Red | 0.02150000 | *<0.07* | 0.07360000 | 0.02150000 | 0.02150000 |
| 10 | | | | | | | | | |
| *10.5* | *BrainPhys Basal* | *QC* | *Properties* | *Osmolarity* | *310 mOsmol*/*L* | *280-330 mOsmol*/*L* | 257 *mOsmol*/*L* | *220 mOsmol*/*L* | 315 *mOsmol*/*L* |
| *10.5* | *BrainPhys Basal* | *QC* | *Properties* | *pH* | *7.40000000* | 7.3-7.5 | | | |
| *10.6* | | | | | | | | | |
| 20 | BrainPhys supplements | | Protein (Neurotrophic factors) | Human Brain Derived Neurotrophic Factor (BDNF) | 20 ug/L | *<40 ug*/*L* | 0.0000000 | 0.0000000 | 0.0000000 |
| 20 | BrainPhys supplements | | Protein (Neurotrophic factors) | Glia neurotrophic factor (GDNF) | 20 ug/L | <*40 uglL* | 0.0000000 | 0.0000000 | 0.0000000 |
| 21 | BrainPhys supplements | | Protein | Laminin | ∼1 mg/L | <20mg/L | 0.0000000 | 0.0000000 | 0.0000000 |
| 21 | BrainPhys supplements (B27) # | | Protein | BSA, Fatty acid free Fraction V | B27 | | | | |
| 21 | BrainPhys supplements (B27) # | | Protein | Catalase | B27 | | | | |
| 21 | BrainPhys supplements (B27) # | | Protein | Superoxide Dismutase | B27 | | | | |
| 21 | BrainPhys supplements (B27) # | | Protein | Human Transferrin | B27 | | | | |
| 21 | BrainPhys supplements (N2) | | Protein | Human Transferrin (Holo) | 0.0010000 | *<2mg*/*dt* | 0.0000000 | 0.0000000 | 0.0000000 |
| 21 | BrainPhys supplements (B27) # | | Protein | Human recombinant insulin | B27 | | | | |

| | **BrainPhys medium** | **Ingredient function** | **Ingrdient class** | **Imgredient** | **BrainPhys (CB2) mM** | ***Ranges of BrainsPhys (CB2) mM*** | **Neurobasal-A (mM)** | **Neurobasal (mM)** | **DMEM/F12, Glutamax #10565 (mM)** |
|---|---|---|---|---|---|---|---|---|---|
| 21 | BrainPhys supplements (N2) | | Protein | Insulin Recombinant Full Chain | 0.0008610 | *<1 uU*/*ml* | 0.0000000 | 0.0000000 | 0.0000000 |
| 22 | BrainPhys supplements (B27) # | | Hormone Steroid | Corticosterone | B27 | | | | |
| 22 | BrainPhys supplements (N2 - B27) | ∼Neuroactive | Hormone Steroid | Progesterone | 0.0000200 + B27 | | | | |
| 22.1 | BrainPhys supplements (B27) # | | Hormone Thyroid | T3 (triodo-I-thyronine) | B27 | | | | |
| 23 | BrainPhys supplements (B27) # | | Essential fatty acid | Linoleic Acid | B27 | *<0.00015* | 0.0000000 | 0.0000000 | 0.0001500 |
| 23 | BrainPhys supplements (B27) # | | Essential fatty acid | Linolenic Acid | B27 | | | | |
| 23 | BrainPhys supplements | | Lipid | Cholesterol | 5 ug/ml | *<15 ug*/*ml* | 0.0000000 | 0.0000000 | 0.0000000 |
| 24 | BrainPhys supplements # | | Vitamin | Ascorbic acid (Vit C) | 0.25 | *<0.5* | 0.0000000 | 0.0000000 | 0.0000000 |
| 24 | BrainPhys supplements (B27) # | | Vitamin | Biotin (B7) | B27 | ***<00001*** | 0.0000000 | 0.0000000 | 0.0000143 |
| 24 | BrainPhys supplements (B27) # | | Vitamin | DL Alpha Tocopherol Acetate | B27 | | | | |

| | **BrainPhys medium** | **Ingredient function** | **Ingredient class** | **Ingredient** | **BrainPhys (CB2) mM** | ***Ranges of BrainPhys (CB2) mM*** | **Neurobasal-A (mM)** | **Neurobasal (mM)** | **DMEM/F12, Glutamax #10565 (mM)** |
|---|---|---|---|---|---|---|---|---|---|
| 24 | BrainPhys supplements (B27) # | | Vitamin | DL Alpha-Tocopherol | B27 | | | | |
| 24 | BrainPhys supplements (B27) # | | Vitamin | Vitamin A (Retinoic acid) | B27 | | | | |
| 25 | BrainPhys supplements (N2 - B27) # | | Sulfate mineral | Selenite | 0.00003 + B27 | | | | |
| 26 | BrainPhys supplements (N2 - B27) # | ∼Neuroactive | Organic chemical compound | Putrescine 2HCl | 0.1001 +B27 | *<0.15* | 0.0000000 | 0.0000000 | 0.0005030 |
| 27 | BrainPhys supplements (B27) # | | Monosacharide | D-Galactose | B27 | | | | |
| 28 | BrainPhys supplements | | Nucleotide | Dibutyril cAMP sodium salt | 0.0010000 | *<0.002* | 0.0000000 | 0.0000000 | 0.0000000 |
| 30 | | | | | | | | | |
| | | | | | | | | | |
| | | | | | | | | | |
| | | | | | | | | | |
| 30.1 | EXCLUDED | Neuroactive | Supplements | serum | 0 | *0.0000000* | | | |
| 40 | | | | | | | | | |
| 40.1 | EXCLUDED | | Other components | Hypoxanthine Na | 0 | *<0.015* | 0.0000000 | 0.0000000 | 0.0150000 |
| 40.1 | EXCLUDED | | Organosulfur compound | Lipoic Acid | 0 | <*0.0006* | 0.0000000 | 0.0000000 | 0.0005100 |

| | **BrainPhys medium** | **Ingredient function** | **Ingredient class** | **Imgredient** | **BrainPhys (CB2) mM** | ***Ranges of BrainPhys (CB2) mM*** | **Neurobasal-A (mM)** | **Neurobasal (mM)** | **DMEM/F12, Glutamax #10565 (mM)** |
|---|---|---|---|---|---|---|---|---|---|
| 40.1 | EXCLUDED | | DNA nucleoside | Thymidine | 0 | *<0.002* | 0.0000000 | 0.0000000 | 0.0015100 |

| | | | | | | | | | |
|---|---|---|---|---|---|---|---|---|---|
| o = Reduced in BrainPhys Opto a = Excluded in BrainPhys without Hepes # = May be removed for cytotoxicity evaluation | | | | | | | | | |

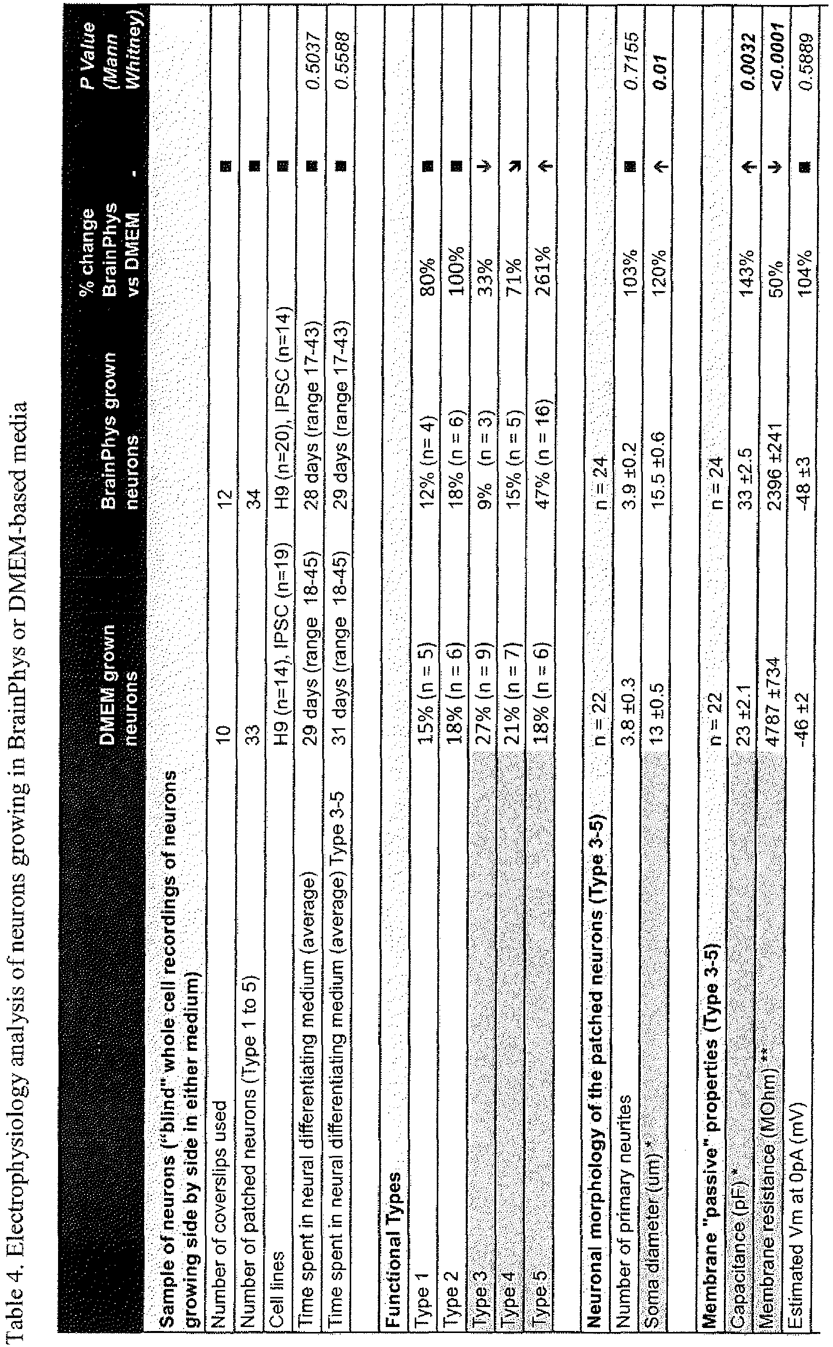

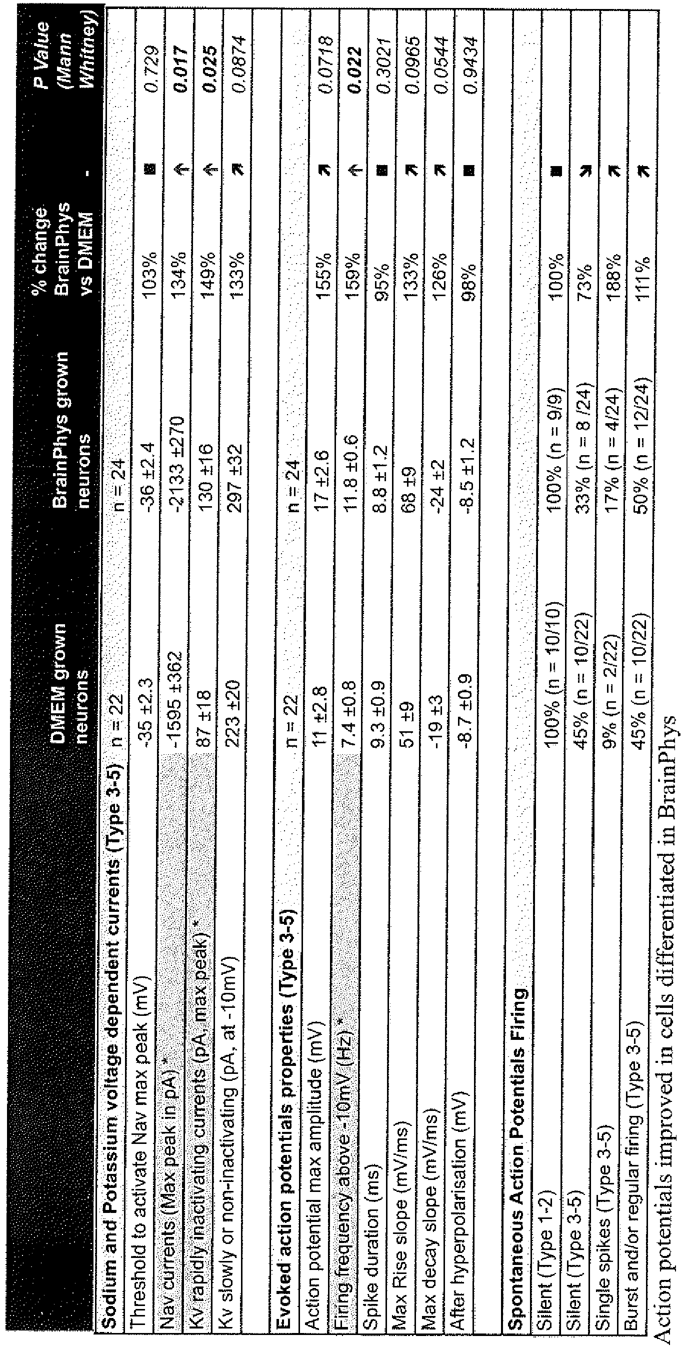

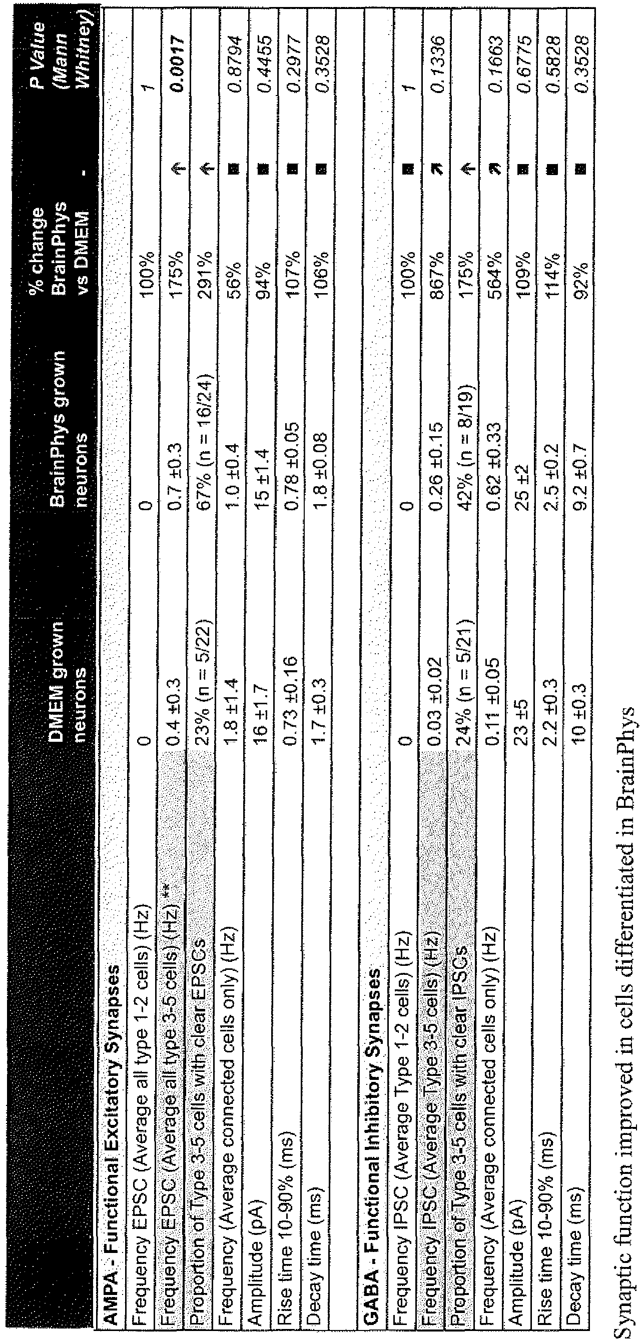

## Claims

1. A cell medium comprising:
(a) Sodium chloride at a concentration of between 70 and 150 mM;
(b) a neuroactive inorganic salt at a concentration of between 0.000001 and 10 mM, selected from the group consisting of Potassium Chloride, Calcium Chloride, Magnesium Sulfate, Magnesium Chloride, Ferric Nitrate, Zinc sulfate, Cupric sulfate, Ferric sulfate, and combinations thereof;
(c) Glycine at a concentration of between 0.0001 and 0.05 mM;
(d) L-alanine at a concentration of between 0.0001 and 0.05 mM; and
(e) L-serine at a concentration of between 0.001 and 0.03 mM,
wherein (a) through (e) are each present at a concentration that maintains the survival and neural functionality of a neural cell cultured in the medium.

2. The cell medium of claim 1, further comprising one or more of
(i) L-aspartic acid at a concentration of between 0.00001 and 0.003 mM;
(ii) L-glutamic acid at a concentration of between 0.00001 and 0.02 mM; and
(iii) a pH modulating agent, wherein the agent is an inorganic salt.

3. The cell medium of claim 2, wherein
(i) the inorganic salt is at a concentration of between 0.001 and 1 mM, and wherein the inorganic salt is selected from the group consisting of Sodium Phosphate dibasic, Sodium Phosphate monobasic, and combinations thereof; and/or
(ii) the inorganic salt is at a concentration of between 1 and 35 mM, and wherein the inorganic salt is Sodium Bicarbonate.

4. The cell medium of claim 1, further comprising one or more of
(i) one or more amino acid, wherein each amino acid is at a concentration of between 0.001 and 1 mM;
(ii) one or more vitamin, wherein each vitamin is present at a concentration of between 0.00001 and 1 mM;
(iii) a supplemental agent selected from the group consisting of a protein, neurotrophic factor, steroid, hormone, fatty acid, lipid, vitamin, sulfate mineral, organic chemical compound, monosaccharide, nucleotide, and combinations thereof;
(iv) an energetic substrate at a concentration of between 0.1 and 5 mM; and
(v) a light sensitive agent.

5. The cell medium of claim 4, wherein the one or more amino acid is selected from the group consisting of L-Alanyl-L-Glutamine, L-Arginine hydrochloride, L-Asparagine-H2O, L-Cysteine hydrochloride-H2O, L-Cystine 2HCl, L-Histidine hydrochloride-H2O, L-Isoleucine, L-Leucine, L-Lysine hydrochloride, L-Methionine, L-Phenylalanine, L-Proline, L-Threonine, L-Tryptophan, L-Tyrosine disodium salt dihydrate, L-Valine, and combinations thereof.

6. The cell medium of claim 4, wherein the one or more vitamin is selected from the group consisting of Choline chloride, D-Calcium pantothenate (B5), Folic Acid (B9), i-Inositol, Niacinamide (B3), Pyridoxine hydrochloride, Thiamine hydrochloride, Vitamin B12 (cyanocobalamin), Riboflavin (B2), and combinations thereof.

7. The cell medium of claim 4, wherein the energetic substrate is selected from the group consisting of sugar, sodium pyruvate, and combinations thereof.

8. The cell medium of claim 4, wherein the light sensitive agent is
(i) Riboflavin (B2) at a concentration of between 0.0001 and 0.0006 mM; and/or
(ii) HEPES at a concentration of between 1 and 10 mM.

9. The cell medium of claim 1, wherein
(i) the cell medium does not comprise serum; and/or
(ii) the osmolarity of the medium is between 280 and 330 Osm/L.

10. A method of culturing a neuronal cell comprising contacting a neuronal cell with a medium of any one of claims 1-9.

11. The method of claim 10, wherein the neuronal cell is a primary neuronal cell.

12. The method of claim 10, wherein said neuronal cell is an iPSC-derived neuronal cell.

13. A composition comprising a neuronal cell cultured in a medium of any one of claims 1-9.

## Patentansprüche

1. Zellmedium, umfassend:
(a) Natriumchlorid bei einer Konzentration von zwischen 70 und 150 mM;
(b) ein neuroaktives anorganisches Salz bei einer Konzentration von zwischen 0,000001 und 10 mM, ausgewählt aus der Gruppe, bestehend aus Kaliumchlorid, Calciumchlorid, Magnesiumsulfat, Magnesiumchlorid, Eisen(III)nitrat, Zinksulfat, Kupfer(II)sulfat, Eisen(III)sulfat und Kombinationen davon;
(c) Glycin bei einer Konzentration von zwischen 0,0001 und 0,05 mM;
(d) L-Alanin bei einer Konzentration von zwischen 0,0001 und 0,05 mM; und
(e) L-Serin bei einer Konzentration von zwischen 0,001 und 0,03 mM,
wobei (a) bis (e) jeweils bei einer Konzentration vorliegen, die das Überleben und neurale Funktionalität einer neuralen Zelle, die in dem Medium gezüchtet wird, aufrecht erhält.

2. Zellmedium nach Anspruch 1, weiter umfassend eine oder mehrere von
(i) L-Asparaginsäure bei einer Konzentration von zwischen 0,00001 und 0,003 mM;
(ii) L-Glutaminsäure bei einer Konzentration von zwischen 0,00001 und 0,02 mM; und
(iii) einem pH modulierenden Mittel, wobei das Mittel ein anorganisches Salz ist.

3. Zellmedium nach Anspruch 2, wobei
(i) das anorganische Salz bei einer Konzentration von zwischen 0,001 und 1 mM vorliegt, und wobei das anorganische Salz ausgewählt ist aus der Gruppe, bestehend aus zweibasigem Natriumphosphat, einbasigem Natriumphosphat und Kombinationen davon; und/oder
(ii) das anorganische Salz bei einer Konzentration von zwischen 1 und 35 mM vorliegt, und wobei das anorganische Salz Natriumbicarbonat ist.

4. Zellmedium nach Anspruch 1, weiter umfassend eine oder mehrere von
(i) einer oder mehreren Aminosäure(n), wobei jede Aminosäure bei einer Konzentration von zwischen 0,001 und 1 mM vorliegt;
(ii) einem oder mehreren Vitamin(en), wobei jedes Vitamin bei einer Konzentration von zwischen 0,00001 und 1 mM vorliegt;
(iii) einem Ergänzungsmittel, ausgewählt aus der Gruppe, bestehend aus einem Protein, neurotrophem Faktor, Steroid, Hormon, Fettsäure, Lipid, Vitamin, Sulfatmineral, organischer chemischer Verbindung, Monosaccharid, Nucleotid und Kombinationen davon;
(iv) einem energetischen Substrat bei einer Konzentration von zwischen 0,1 und 5 mM; und
(v) einem lichtempfindlichen Mittel.

5. Zellmedium nach Anspruch 4, wobei die eine oder mehreren Aminosäure(n) ausgewählt ist aus der Gruppe, bestehend aus L-Alanyl-L-glutamin, L-Argininhydrochlorid, L-Asparagin-H2O, L-Cysteinhydrochlorid-H2O, L-Cystin 2HCl, L-Histidinhydrochlorid-H2O, L-Isoleucin, L-Leucin, L-Lysinhydrochlorid, L-Methionin, L-Phenylalanin, L-Prolin, L-Threonin, L-Tryptophan, L-Tyrosindinatriumsalzdihydrat, L-Valin und Kombinationen davon.

6. Zellmedium nach Anspruch 4, wobei das eine oder mehrere Vitamin(e) ausgewählt ist aus der Gruppe, bestehend aus Cholinchlorid, D-Calciumpantothenat (B5), Folsäure (B9), i-Inosit, Niacinamid (B3), Pyridoxinhydrochlorid, Thiaminhydrochlorid, Vitamin B12 (Cyanocobalamin), Riboflavin (B2) und Kombinationen davon.

7. Zellmedium nach Anspruch 4, wobei das energetische Substrat ausgewählt ist aus der Gruppe, bestehend aus Zucker, Natriumpyruvat und Kombinationen davon.

8. Zellmedium nach Anspruch 4, wobei das lichtempfindliche Mittel ist
(i) Riboflavin (B2) bei einer Konzentration von zwischen 0,0001 und 0,0006 mM; und/oder
(ii) HEPES bei einer Konzentration von zwischen 1 und 10 mM.

9. Zellmedium nach Anspruch 1, wobei
(i) das Zellmedium kein Serum umfasst; und/oder
(ii) die Osmolarität des Mediums zwischen 280 und 330 Osm/l liegt.

10. Verfahren zum Züchten einer neuronalen Zelle, umfassend in Kontakt bringen einer neuronalen Zelle mit einem Medium nach einem der Ansprüche 1-9.

11. Verfahren nach Anspruch 10, wobei die neuronale Zelle eine primäre neuronale Zelle ist.

12. Verfahren nach Anspruch 10, wobei die neuronale Zelle eine iPSC-abgeleitete neuronale Zelle ist.

13. Zusammensetzung, umfassend eine neuronale Zelle, gezüchtet in einem Medium nach einem der Ansprüche 1-9.

## Revendications

1. Milieu cellulaire comprenant:
(a) du chlorure de sodium à une concentration comprise entre 70 et 150 mM ;
(b) un sel inorganique neuroactif à une concentration comprise entre 0,000001 et 10 mM, choisi dans le groupe constitué par le chlorure de potassium, le chlorure de calcium, le sulfate de magnésium, le chlorure de magnésium, le nitrate ferrique, le sulfate de zinc, le sulfate cuivrique, le sulfate ferrique et des combinaisons de ceux-ci ;
(c) de la glycine à une concentration comprise entre 0,0001 et 0,05 mM ;
(d) de la L-alanine à une concentration comprise entre 0,0001 et 0,05 mM ; et
(e) de la L-sérine à une concentration comprise entre 0,001 et 0,03 mM,
dans lequel (a) à (e) sont chacun présents à une concentration qui maintient la survie et la fonctionnalité neurale d'une cellule neurale cultivée dans le milieu.

2. Milieu cellulaire selon la revendication 1, comprenant en outre un ou plusieurs parmi :
(i) l'acide L-aspartique à une concentration comprise entre 0,00001 et 0,003 mM ;
(ii) l'acide L-glutamique à une concentration comprise entre 0,00001 et 0,02 mM ; et
(iii) un agent de modulation du pH, l'agent étant un sel inorganique.

3. Milieu cellulaire selon la revendication 2, dans lequel
(i) le sel inorganique est à une concentration comprise entre 0,001 et 1 mM, et le sel inorganique étant choisi dans le groupe constitué par le phosphate de sodium dibasique, le phosphate de sodium monobasique et des combinaisons de ceux-ci ; et/ou
(ii) le sel inorganique est à une concentration comprise entre 1 et 35 mM, et le sel inorganique étant le bicarbonate de sodium.

4. Milieu cellulaire selon la revendication 1, comprenant en outre un ou plusieurs parmi :
(i) un ou plusieurs acides aminés, où chaque acide aminé est à une concentration comprise entre 0,001 et 1 mM ;
(ii) une ou plusieurs vitamines, où chaque vitamine est présente à une concentration comprise entre 0,00001 et 1 mM ;
(iii) un agent supplémentaire choisi dans le groupe consistant en une protéine, un facteur neurotrophique, un stéroïde, une hormone, un acide gras, un lipide, une vitamine, un sulfate minéral, un composé chimique organique, un monosaccharide, un nucléotide et des combinaisons de ceux-ci ;
(iv) un substrat énergétique à une concentration comprise entre 0,1 et 5 mM ; et
(v) un agent photosensible.

5. Milieu cellulaire selon la revendication 4, dans lequel le ou les acides aminés sont choisis dans le groupe constitué par la L-alanyl-L-glutamine, le chlorhydrate de L-arginine, la L-asparagine-H20, le chlorhydrate de cystéine-H2O, la L- Cystine 2HCl, le chlorhydrate de L-histidine-H2O, la L-isoleucine, la L-leucine, le chlorhydrate de L-lysine, la L-méthionine, la L-phénylalanine, la L-proline, la L-thréonine, le L tryptophane, le dihydrate de sel disodique de L-tyrosine, la L- Valine et des combinaisons de ceux-ci.

6. Milieu cellulaire selon la revendication 4, dans lequel la ou les vitamines sont choisies dans le groupe constitué par le chlorure de choline, le pantothénate de D-calcium (B5), l'acide folique (B9), le i-Inositol, la niacinamide (B3), le chlorhydrate de pyridoxine, le chlorhydrate de thiamine, la vitamine B12 (cyanocobalamine), la riboflavine (B2) et des combinaisons de ceux-ci.

7. Milieu cellulaire selon la revendication 4, dans lequel le substrat énergétique est choisi dans le groupe constitué par le sucre, le pyruvate de sodium et des combinaisons ceux-ci.

8. Milieu cellulaire selon la revendication 4, dans lequel l'agent photosensible est :
(i) la riboflavine (B2) à une concentration comprise entre 0,0001 et 0,0006 mM ; et/ou
(ii) l'HEPES à une concentration comprise entre 1 et 10 mM.

9. Milieu cellulaire selon la revendication 1, dans lequel
(i) le milieu cellulaire ne comprend pas de sérum ; et/ou
(ii) l'osmolarité du milieu est comprise entre 280 et 330 Osm/L.

10. Procédé de culture d'une cellule neuronale comprenant la mise en contact d'une cellule neuronale avec un milieu selon l'une quelconque des revendications 1-9.

11. Procédé selon la revendication 10, dans lequel la cellule neuronale est une cellule neuronale primaire.

12. Procédé selon la revendication 10, dans lequel ladite cellule neuronale est une cellule neuronale dérivée d'iPSC.

13. Composition comprenant une cellule neuronale cultivée dans un milieu selon l'une quelconque des revendications 1-9.
